# EUROPEAN PATENT SPECIFICATION

(11) **EP 2 249 410 B1**
(45) Date of publication and mention of the grant of the patent: **02.12.2015**
(21) Application number: 09715469.4
(22) Date of filing: 23.02.2009
(51) Int. Cl.: H01L 51/50, C09K 11/06, C07F 5/02, C07C 211/54, C07C 211/56, H05B 33/14

(54) **ORGANIC ELECTROLUMINESCENT ELEMENT, AND MANUFACTURING METHOD AND USES THEREFOR**
ORGANISCHES ELEKTROLUMINESZENZELEMENT SOWIE VERFAHREN ZU SEINER HERSTELLUNG UND VERWENDUNG
ÉLÉMENT ÉLECTROLUMINESCENT ORGANIQUE, ET PROCÉDÉ DE FABRICATION ET UTILISATIONS APPARENTÉS

(30) Priority: 25.02.2008 JP 2008043288
(43) Date of publication of application: 10.11.2010
(73) Proprietor: Samsung Electronics Co., Ltd., Suwon-si, Gyeonggi-do 443-772 (KR)
(72) Inventor: FUKUCHI, Yousuke, Chiba-shi Chiba 267-0056 (JP); IGARASHI, Takeshi, Chiba-shi Chiba 267-0056 (JP)
(74) Representative: Greene, Simon Kenneth
(86) International application number: PCT/JP2009/053139
(87) International publication number: WO 2009/107574

(56) References cited:
- WO-A1-00/14174
- WO-A1-2005/094133
- WO-A1-2006/112265
- WO-A1-2006/134720
- WO-A1-2007/026846
- WO-A2-2007/079103
- JP-A- 11 307 255
- JP-A- 2000 063 335
- JP-A- 2001 196 174
- JP-A- 2002 175 879
- JP-A- 2002 184 575
- JP-A- 2003 123 983
- JP-A- 2003 142 269
- JP-A- 2003 234 192
- JP-A- 2006 203 172
- JP-A- 2007 070 282
- JP-A- 2007 070 282
- JP-A- 2007 084 439
- JP-A- 2007 109 988
- JP-A- 2007 134 677
- US-A1- 2006 036 114
- US-A1- 2007 087 219
- US-A1- 2007 167 588
- UCHIDA M ET AL: "UNDOPING TYPE OF HIGHLY EFFICIENT ORGANIC LIGHT EMITTING DIODES", JOURNAL OF PHOTOPOLYMER SCIENCE AND TECHNOLOGY, TECHNICAL ASSOCIATION OF PHOTOPOLYMERS JAPAN, JP, vol. 14, no. 2, 1 January 2001 (2001-01-01), pages 305-310, XP001041358, ISSN: 0914-9244

## Description

### FIELD OF THE INVENTION

The present invention relates to organic electroluminescence devices, processes for producing the same, and uses of the devices.

### BACKGROUND OF THE INVENTION

Since C. W. Tang et al. of Eastman Kodak Company invented organic electroluminescence devices (hereinafter, also the organic EL devices) achieving high brightness (Non-patent Document 1) in 1987, there have been rapid progresses in the development of materials for the organic EL devices and the improvements in device structures. Practical use of the organic EL devices has started recently in displays of car audio systems and cellular phones. In order to expand the use of the organic EL (electroluminescence) devices, studies have been carried out to develop materials that provide higher luminous efficiency and durability or to develop full color displays.

The current materials for the organic EL devices are low-molecular materials and high-molecular materials. Because the device manufacturing generally involves lamination, the use of low-molecular materials causes complexity in production steps for the organic EL devices. On the other hand, high-molecular materials have a difficult purification problem and consequently provide lower emission properties and life of the devices than achieved with the low-molecular materials.

To solve these problems, active researches have been made on materials having an intermediate molecular weight between the low-molecular materials and the high-molecular materials.

For example, Patent Document 1 discloses compounds represented by Formula (I) below. The compounds are mainly used as materials for hole transport layers of organic EL devices.

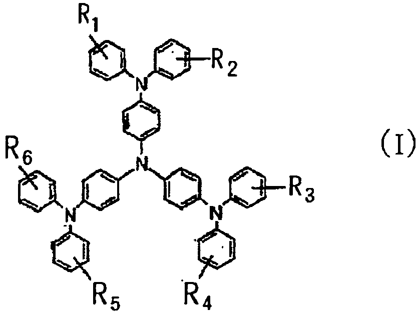

In Formula (I), R₁, R₂, R₃, R₄, R₅ and R₆ are each a hydrogen atom, an alkyl group, an alkoxy group, a 3-phenyl group, a phenoxy group, an arylamino group or a diarylamino group, and at least one of R₁, R₂, R₃, R₄, R₅ and R₆ is a 3-phenyl group, an arylamino group or a diarylamino group.

Patent Document 2 discloses arylamine compounds of Formula (II) below having a molecular weight of 1500 to 6000. The arylamine compounds are used as materials for thin layers such as hole injection layers and hole transport layers in organic EL devices.

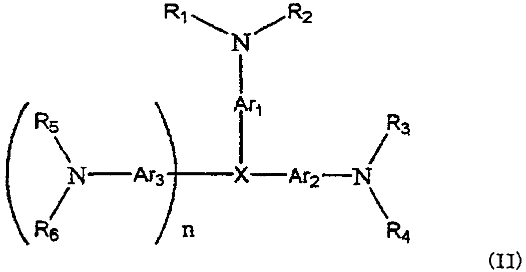

In Formula (II), X represents a single bond, a carbon atom or a nitrogen atom, Ar₁, Ar₂ and Ar₃ are the same as one another and represent phenyl groups, biphenylyl groups or terphenyl groups, R₁, R₂, R₃, R₄, R₅ and R₆ are each independently an aryl group, the aryl group(s) may form a triphenylamine structure by being substituted with a diarylamino group, the terminal aryl group(s) may form a sequence of triphenylamine structures by being substituted with diarylamino groups, and the letter n indicates 0 or 1.

Patent Document 3 discloses that compounds having an N-phenylcarbazole structure and a triphenylamine structure, for example as illustrated below, are used as charge transport materials for organic EL devices.

Boron derivatives that are used in electroluminescent layers for OLEDs have already been shown in US2006/0036114, US2007/0167588, US2007/0087219 and by UCHIDA M ET AL: "UNDOPING TYPE OF HIGHLY EFFICIENT ORGANIC LIGHT EMITTING DIODES", JOURNAL OF PHOTOPOLYMER SCIENCE AND TECHNOLOGY, TECHNICAL ASSOCIATION OF PHOTOPOLYMERS JAPAN, JP,vol. 14, no. 2, 1 January 2001 [0009]

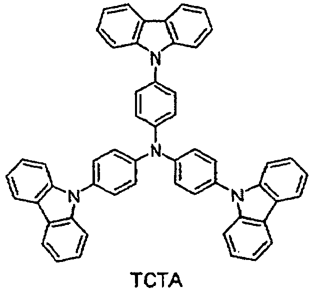

However, there have been developed no materials having sufficient luminous efficiency and durability. In particular, the application of organic EL devices in displays requires materials that have high luminous efficiency and superior durability permitting stable and continuous drive of the devices.
[Patent Document 1] JP-A-H10-284252
[Patent Document 2] JP-A-2007-84439
[Patent Document 3] JP-A-2007-110093
[Non-patent Document 1] Appl. Phys. Lett., 1987, Vol. 51, p. 913

### SUMMARY OF THE INVENTION

It is an obj ect of the present invention to provide organic electroluminescence devices having excellent luminous efficiency and durability, production processes therefor and uses of the devices.

The present inventors studied diligently to solve the problems in the background art as described above. They have then found that excellent luminous efficiency and durability are achieved by organic electroluminescence devices as defined in claims 1-4, and by a process for producing organic electroluminescence devices according to claim 5.

In the present invention, a hole transporting compound, an electron transporting compound and an emitting compound form a single layer as a mixture. Accordingly, organic electroluminescence devices having excellent luminous efficiency and durability are manufactured easily. Patent Documents 1 to 3 do not explicitly describe that the above three kinds of compounds are mixed together and form a single layer.

### ADVANTAGEOUS EFFECTS OF THE INVENTION

The organic electroluminescence devices of the invention have excellent luminous efficiency and durability and are suitably used in applications such as displays and illuminations. According to the processes for producing organic electroluminescence devices of the invention, organic electroluminescence devices having excellent luminous efficiency and durability are manufactured easily.

### BRIEF DESCRIPTION OF THE DRAWING

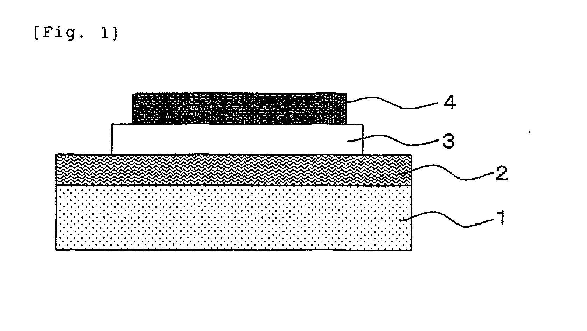

Fig. 1 is a cross-sectional view of an organic electroluminescence device according to an embodiment of the invention.

- 1:: transparent substrate
- 2:: anode
- 3:: emitting layer
- 4:: cathode

### PREFERRED EMBODIMENTS FOR CARRYING OUT THE INVENTION

### (Organic electroluminescence compounds)

The organic electroluminescence devices of the invention have a layer formed from organic electroluminescence compounds such as hole transporting compounds, electron transporting compounds and emitting compounds. The organic electroluminescence compounds will be described below.

### (Hole transporting compounds)

The hole transporting compounds used in the invention have 4 to 8 arylamine structures in the molecule.

When the hole transporting compounds have the above number of arylamine structures in the molecule, sufficient hole transporting properties are achieved. Further, such hole transporting compounds can be purified easily, and the purity thereof can be increased. The increased purity tends to lead to higher luminous efficiency and durability of the obtained organic electroluminescence devices. Only one arylamine structure in the molecule does not provide sufficient hole transporting properties. If the hole transporting compounds have 11 or more arylamine structures, the purification of the compounds tends to be difficult.

In the invention, the "arylamine compounds" refer to amine compounds that have an aryl group directly bonded to the nitrogen atom of the amine compounds. Herein, the aryl groups mean "residues of aromatic hydrocarbons after removal of one hydrogen atom". (That is, phenyl, naphthyl and the like are collectively referred to as the aryl groups. See Hyojun Kagaku Yogo Jiten (Standard Dictionary of Chemical Terms), pocket edition, March 20, 2000, fifth printing, edited by The Chemical Society of Japan, published from MARUZEN Co., Ltd., p. 19.)

In the invention, the arylamine structure refers to a structure represented by Formula (1) below:

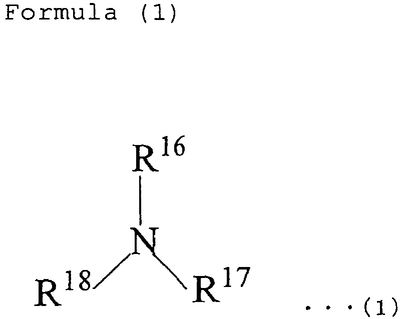

In Formula (1), R¹⁶, R¹⁷ and R¹⁸ are each independently an alkyl group, an aralkyl group, an aryl group or a residue of an alkyl, aralkyl or aryl group after removal of one hydrogen atom therefrom, and at least one of R¹⁶, R¹⁷ and R¹⁸ is an aryl group or a residue of an aryl group after removal of one hydrogen atom therefrom.

The alkyl groups include methyl, ethyl, n-propyl and iso-propyl groups. The aralkyl groups include benzyl and phenethyl groups. The aryl groups include phenyl, naphthyl, biphenyl, fluorenyl, terphenyl, phenanthrenyl, pyrenyl, heptalenyl, azulenyl, phenalenyl, anthryl, thienyl, bithienyl, thiopyranyl, furanyl, pyranyl, pyridyl, pyrrolyl, imidazolyl, pyrazolyl, pyrazinyl, pyrimidinyl, pyridazinyl, purinyl and triazinyl groups. Examples of these groups are not limited to those described above.

In a particularly preferred embodiment, R¹⁶, R¹⁷ and R¹⁸ are all aryl groups.

In the invention, a structural unit represented by Formula (1) is counted as one arylamine structure. In the counting of the structures, R¹⁶, R¹⁷ and R¹⁸ in Formula (1) may be shared between arylamine structures.

When the above rule is applied to compounds 1 to 9 described later, the compounds 2, 5 and 9 have four arylamine structures, the compounds 3, 6 and 8 have six arylamine structures, and the compounds 1, 4 and 7 have seven arylamine structures.

The hole transporting compounds in the invention preferably have at least 10, more preferably 10 to 28, and still more preferably 12 to 24 benzene ring structures in the molecule. When the hole transporting compounds have the benzene ring structures in this range, amorphous, thermally stable thin layers can be formed.

The hole transporting compounds in the invention have a molecular weight in the range of 800 to 5000, more preferably 1000 to 4000, and still more preferably 1000 to 2000. The hole transporting compounds having this molecular weight provide advantages that amorphous, thermally stable thin layers are formed, the compounds tend to show high solubility in solvents and are suited for forming the layer by coating, and the purity of the compounds can be increased by sublimation purification. If the molecular weight of the hole transporting compounds is below 800, the compounds have so high tendency to crystallize that the organic layer containing the compound may be crystallized during the film formation. If the molecular weight exceeds 5000, the compounds may be less soluble in solvents and the purification of the compounds by sublimation may be difficult.

The hole transporting compounds in the invention preferably have a glass transition temperature Tg in the range of 80 to 300°C, more preferably 80 to 250°C, and still more preferably 100 to 200°C. The hole transporting compounds having this glass transition temperature Tg provide advantages that satisfactory amorphous thin layers are formed and the amorphous thin layers have high thermal stability.

The value of the glass transition temperature Tg in the invention is that determined by DSC as follows.

### (DSC procedures)

The differential scanning calorimeter used is DSC 220 manufactured by Seiko Instruments Inc. A 5 mg sample is placed in an aluminum container and the container is tightly closed. The sample is heated from room temperature to 300°C at a temperature increasing rate of 10°C/min, held at the temperature for 5 minutes, and cooled to 30°C at a temperature decreasing rate of 10°C/min. Immediately thereafter, the sample is heated to 300°C at a rate of 10°C/min and then cooled to 30°C at a rate of 10°C/min, and this procedure is repeated two times.

Examples of the hole transporting compounds include, but are not limited to, compounds 1 to 9 illustrated below.

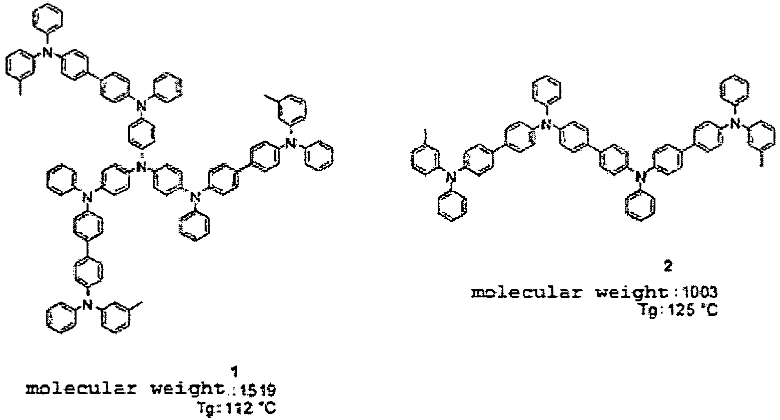

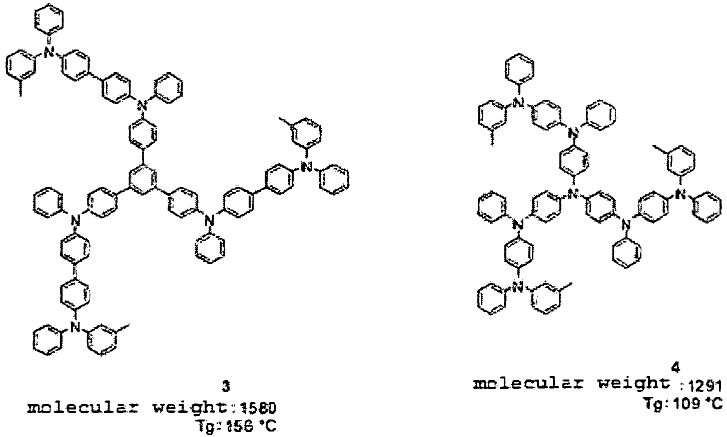

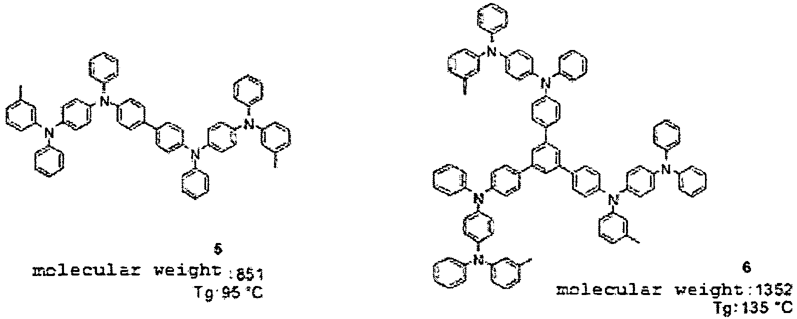

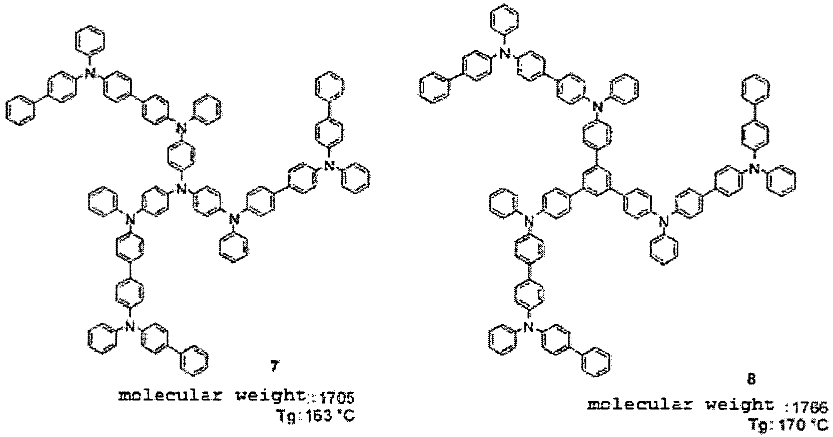

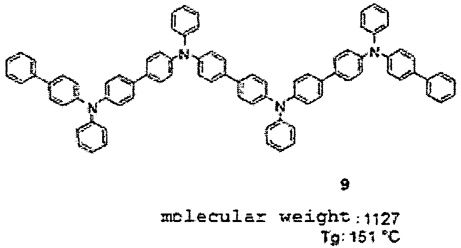

The hole transporting compounds 1 to 9 have sufficient hole transporting properties, and the organic electroluminescence devices manufactured using these compounds achieve high durability.

### (Phosphorescent compounds)

The emitting compounds in the invention are preferably phosphorescent compounds. Preferred phosphorescent compounds are transition metal complexes. The transition metals in the transition metal complexes are metal atoms in the first transition series from atomic number 21 Sc to atomic number 30 Zn, the second transition series from atomic number 39 Y to atomic number 48 Cd, and the third transition series from atomic number 72 Hf to atomic number 80 Hg. Of the transition metal complexes of these metals, palladium complexes, osmium complexes, iridium complexes, platinum complexes and gold complexes are preferred, iridium complexes and platinum complexes are more preferred, and iridium complexes are most preferred.

Specific examples include the following compounds (E-1) to (E-49):

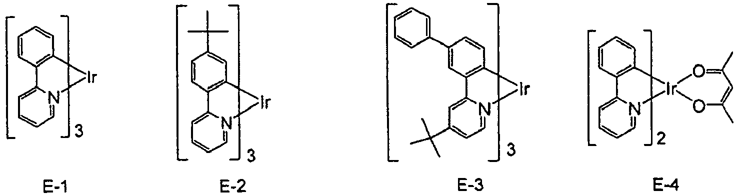

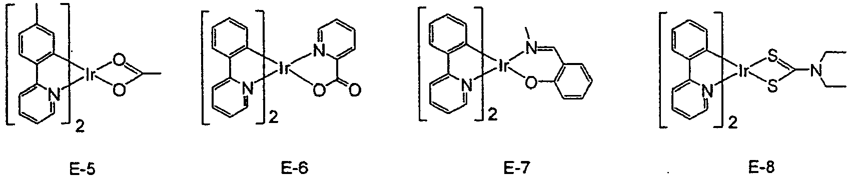

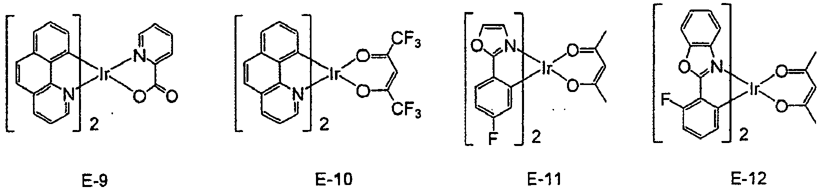

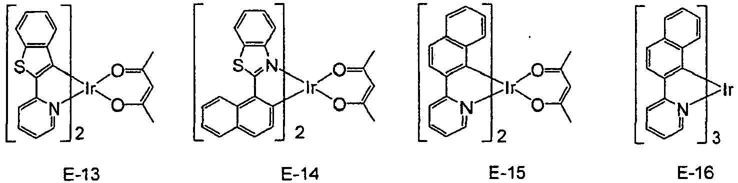

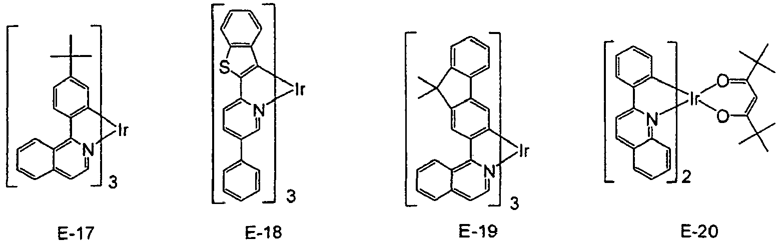

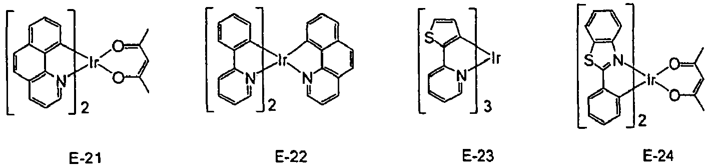

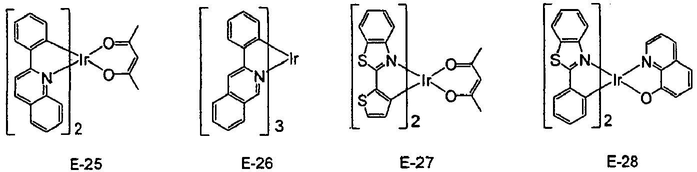

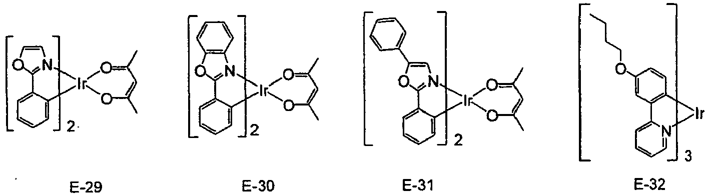

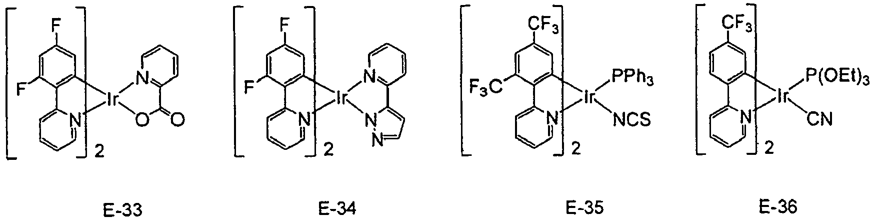

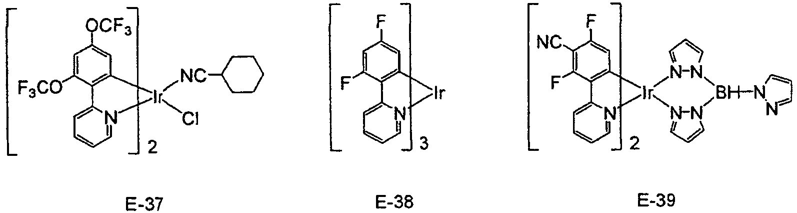

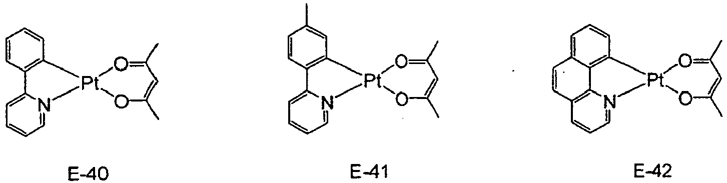

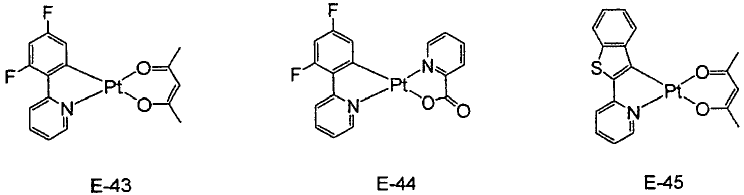

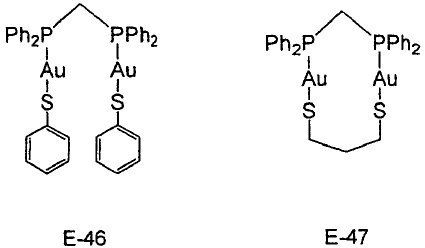

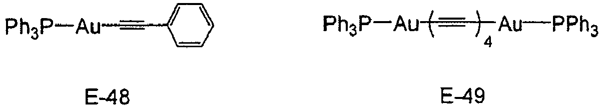

In Formulae (E-35) and (E-46) to (E-49), Ph represents a phenyl group.

### (Electron transporting compounds)

The electron transporting compounds in the invention have a structure represented by Formula (a) below. The use of the electron transporting compounds having a structure of Formula (a) tends to provide high luminous efficiency and durability of the obtained organic electroluminescence devices.

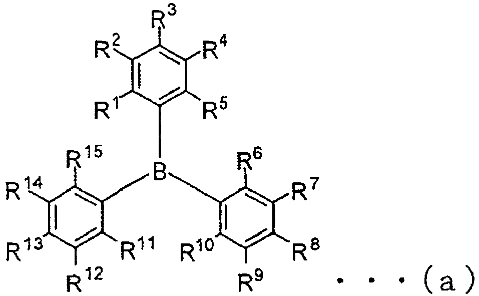

In Formula (a), R¹ to R¹⁵ are each independently a hydrogen atom, a halogen atom, a cyano group, an amino group, a C1-12 alkyl group, a C1-12 alkoxy group, an aryloxy group, an aryl group or a heterocyclic group, and neighboring substituent groups of R¹ to R¹⁵ that are on the same phenyl group may be bonded together to form a condensed ring.

The halogen atoms include fluorine, chlorine, bromine and iodine.

The C1-12 alkyl groups include methyl, ethyl, propyl, isopropyl, butyl, isobutyl, t-butyl, amyl, hexyl, octyl, decyl and dodecyl groups.

The C1-12 alkoxy groups include methoxy, ethoxy, propoxy, isopropoxy, isobutoxy, t-butoxy, hexyloxy, octyloxy, decyloxy and dodecyloxy groups.

The aryloxy groups include phenoxy, 4-methylphenoxy, naphthyloxy and anthranyloxy groups.

The aryl groups include phenyl, biphenyl, terphenyl, tolyl, xylyl, mesityl, naphthyl, anthryl and phenanthryl groups.

The heterocyclic groups include pyridyl, pyrrole, furanyl, thiophene, pyrazole, imidazole, triazole, tetrazole, oxazole, oxadiazole, thiazole, thiadiazole, indole, carbazole, benzofuranyl, benzothiophene, benzimidazole, benzotriazole, benzoxazole, benzothiazole, benzodithiazole and puryl groups.

R¹, R⁵, R⁶, R¹⁰, R¹¹ and R¹⁵ are preferably each a C1-6 alkyl or alkoxy group, and more preferably a methyl group. When R¹, R⁵, R⁶, R¹⁰, R¹¹ and R¹⁵ are such substituent groups, the electron transporting compounds having a structure of Formula (a) tend to be stable to air or water.

The electron transporting compounds may have two or more structural units derived from Formula (a). Particularly preferred electron transporting compounds in the invention are illustrated below:

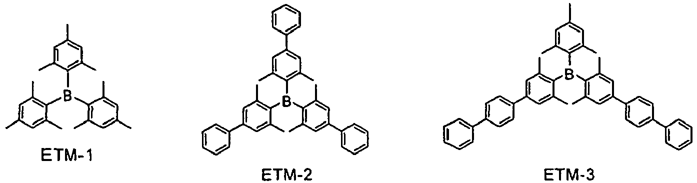

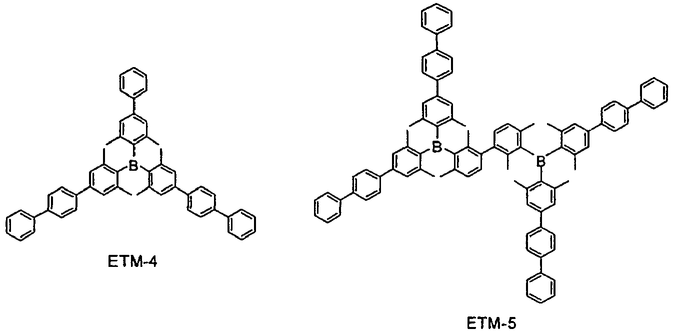

In the invention, the hole transporting compound, the phosphorescent compound and the electron transporting compound are used in the form of a mixture thereof. The organic electroluminescence devices manufactured with the mixture of these compounds tends to achieve high luminous efficiency and durability.

The hole transporting compound, the phosphorescent compound and the electron transporting compound may be mixed together and formed into an organic layer by any methods without limitation. An exemplary method is given below. First, the hole transporting compound, the phosphorescent compound and the electron transporting compound are dissolved in a solvent to give a solution. The solvents used herein are not particularly limited. Examples thereof include chlorinated solvents such as chloroform, methylene chloride and dichloroethane; etherial solvents such as tetrahydrofuran and anisole; aromatic hydrocarbon solvents such as toluene and xylene; ketone solvents such as acetone and methyl ethyl ketone; and ester solvents such as ethyl acetate, butyl acetate and ethyl cellosolve acetate. The solution is then spread on a substrate by a method such as resistance heating evaporation, electron beam deposition, sputtering, or application by spin coating, casting, microgravure coating, gravure coating, bar coating, roll coating, wire bar coating, dip coating, spray coating, screen printing, flexographic printing, off-set printing or inkjet printing. The amount of the solvents depends on the compounds used and layer formation conditions. In the case of spin coating or dip coating, the solution preferably contains the solvent in an amount of 1000 to 100000 parts by mass based on 100 parts by mass of the total of the hole transporting compound, the phosphorescent compound and the electron transporting compound.

In the invention, other electron transporting compounds may be used. Examples thereof include low molecular weight compounds such as quinolinol derivative metal complexes such as Alq₃ (aluminum trisquinolinolate), oxadiazole derivatives, triazole derivatives, imidazole derivatives, triazine derivatives and triarylborane derivatives; and polymer compounds obtained by introducing polymerizable substituent groups to the low molecular weight compounds followed by polymerization, such as poly-PBD as disclosed in JP-A-H10-1665. The additional electron transporting compounds may be used singly or as a mixture of two or more kinds thereof, or differing electron transporting compounds may be laminated.

Examples of the electron transporting compounds include the following compounds:

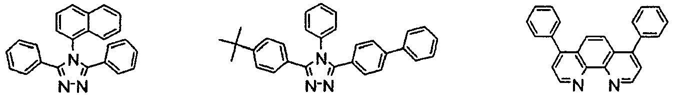

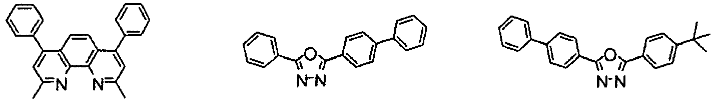

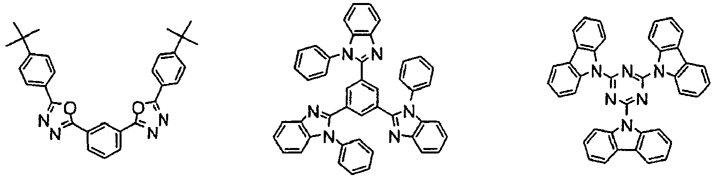

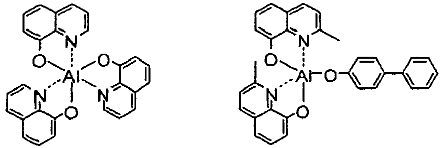

When another electron transport layer is provided, the thickness of the electron transport layer depends on, for example, the conductivity of the electron transport layer, but the thickness is preferably in the range of 1 nm to 5 µm, more preferably 5 nm to 1 µm, and particularly preferably 10 nm to 500 nm.

### (Methods of forming organic electroluminescence compound layers)

The layer containing the organic electroluminescence compounds may be formed by a method such as resistance heating evaporation, electron beam deposition, sputtering, or application by spin coating, casting, microgravure coating, gravure coating, bar coating, roll coating, wire bar coating, dip coating, spray coating, screen printing, flexographic printing, off-set printing or inkjet printing. Resistance heating evaporation and electron beam deposition is usually used for the emitting compounds of low molecular weight. For the emitting compounds having a high molecular weight, the coating methods are usually used such as spin coating, casting, microgravure coating, gravure coating, bar coating, roll coating, wire bar coating, dip coating, spray coating, screen printing, flexographic printing, off-set printing and inkjet printing. After the layer containing the organic electroluminescence compounds is formed by the methods as described above, the compounds may remain as they are or may be polymerized.

### 〈Organic electroluminescence devices)

The organic electroluminescence devices of the invention include a pair of electrodes and one or more organic layers including an emitting layer between the pair of electrodes. One of the organic layer(s) contains the hole transporting compound having 4 to 8 arylamine structures in the molecule and a molecular weight in the range of 800 to 5000, the electron transporting compound having a structure of Formula (a) below and the emitting compound.

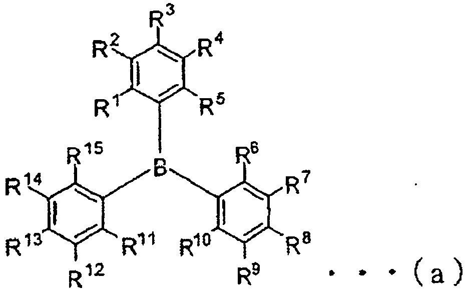

In Formula (a), R¹ to R¹⁵ are each independently a hydrogen atom, a halogen atom, a cyano group, an amino group, a C1-12 alkyl group, a C1-12 alkoxy group, an aryloxy group, an aryl group or a heterocyclic group, and neighboring substituent groups of R¹ to R¹⁵ that are on the same phenyl group may be bonded together to form a condensed ring.

The hole transporting compound preferably has 10 or more benzene ring structures in the molecule.

The hole transporting compound preferably has a glass transition temperature Tg in the range of 80 to 300°C.

### (Anodes)

The anode is a conductive and light transmissive layer as represented by ITO. In the case where the organic emission is obtained through the substrate, the anode should have light transparency. In the case of top emission wherein the organic emission is obtained through the upper electrode, the anode does not need to be optically transparent and any appropriate materials such as metals and metal compounds having a work function of more than 4.1 eV may be used as the anodes. For example, gold, nickel, manganese, iridium, molybdenum, palladium and platinum may be used singly or in combination with one another. In an embodiment, the anode may be selected from metal oxides, metal nitrides, metal selenides and metal sulfides. In another embodiment, the anode may be an ITO anode having good light transparency on which a thin layer of the above metal is formed in a thickness of 1 to 3 nm without deteriorating the light transparency. Such layers may be formed on the surface of anode materials by methods such as electron beam deposition, sputtering, chemical reactions, coating and vacuum deposition. The thickness of the anodes is preferably in the range of 2 to 300 nm.

### (Anode surface treatment)

Prior to the formation of an anode buffer layer or the layer containing the hole transporting compound, the surface of the anode may be treated to improve performances of the layer coated thereon (such as adhesion with the anode substrate, surface smoothness, reduction of hole injection barrier). Such pretreatments include high frequency plasma treatment, sputtering, corona treatment, UV ozone irradiation treatment and oxygen plasma treatment.

### (Anode buffer layers: when Baytron or the like is used)

The anode buffer layers may be prepared by wet processes using coating methods such as spin coating, casting, microgravure coating, gravure coating, bar coating, roll coating, wire bar coating, dip coating, spray coating, screen printing, flexographic printing, off-set printing and inkjet printing.

The compounds used for the film formation by the wet processes are not particularly limited as long as the compounds show good adhesion to the anode surface and the organic EL compounds contained in the layer over the anode buffer layer. It is more preferable to use conventional anode buffers. Examples thereof include conductive polymers such as PEDOT-PSS which is a mixture of poly(3,4)-ethylenedioxythiophene and polystyrenesulfonate and PANI which is a mixture of polyaniline and polystyrenesulfonate. Organic solvents such as toluene and isopropyl alcohol may be added to the conductive polymers. Further, the conductive polymers may contain a third component such as surfactant. The surfactants used herein may contain a group selected from alkyl groups, alkylaryl groups, fluoroalkyl groups, alkylsiloxane groups, sulfates, sulfonates, carboxylates, amides, betaine structures and quaternary ammonium groups. Fluoride-based nonionic surfactants may also be used.

### (Hole blocking layers)

To prevent holes from passing through the emitting layer and to ensure that the holes are recombined with electrons in the emitting layer more efficiently, a hole blocking layer may be provided adjacent to the emitting layer on the cathode side. The hole blocking layer may be produced using a compound that has a highest occupied molecular orbital (HOMO) level deeper than that of the emitting compounds, with examples including triazole derivatives, oxadiazole derivatives, phenanthroline derivatives and aluminum complexes.

Further, an exciton blocking layer may be provided adjacent to the emitting layer on the cathode side to prevent the excitons from being deactivated by the cathode metal. The exciton blocking layer may be formed using a compound that has a triplet excited state energy higher than that of the emitting compounds, with examples including triazole derivatives, phenanthroline derivatives and aluminum complexes.

### (Cathodes)

The cathode materials for the organic electroluminescence devices in the present invention may be known cathode materials that have a low work function and are chemically stable, with examples including Al, MgAg alloy and alloys of Al and alkali metals such as AlLi and AlCa. In view of chemical stability, it is preferable to use materials having a work function of not less than 2.9 eV. These cathode materials may be formed into layers by methods such as resistance heating evaporation, electron beam deposition, sputtering and ion plating. The thickness of the cathodes is preferably in the range of 10 nm to 1 µm, and more preferably 50 to 500 nm.

To lower the barrier for the electron injection from the cathode to the organic layer and thereby to increase the electron injection efficiency, a cathode buffer layer of a metal having a lower work function than the cathode may be interposed between the cathode and the organic layer adjacent to the cathode. Examples of the low work function metals include alkali metals (Na, K, Rb and Cs), alkaline earth metals (Sr, Ba, Ca and Mg) and rare earth metals (Pr, Sm, Eu and Yb). Further, alloys and metal compounds may be used as long as their work functions are lower than that of the cathode. The cathode buffer layers may be produced by methods such as vapor deposition and sputtering. The thickness of the cathode buffer layers is preferably in the range of 0.05 to 50 nm, more preferably 0.1 to 20 nm, and still more preferably 0.5 to 10 nm.

In an embodiment, the cathode buffer layer may be formed of a mixture of the above low work function substance and an electron transporting compound. The electron transporting compounds used herein may be the organic compounds used in the electron transport layers described above. Such cathode buffer layers may be formed by a codeposition method. In the case where such layers can be formed by the coating of a solution, the foregoing methods such as spin coating, dip coating, inkjet printing, printing, spraying and dispenser printing may be adopted. The thickness of such cathode buffer layers is preferably in the range of 0.1 to 100 nm, more preferably 0.5 to 50 nm, and still more preferably 1 to 20 nm. In another embodiment, a conductive polymer layer or a layer of metal oxide, metal fluoride or insulating organic material having an average thickness of not more than 2 nm may be provided between the cathode and the organic layer.

### (Sealing)

After the cathode is prepared, a protective layer for protecting the organic electroluminescence device may be provided. To allow for long and stable use of the organic electroluminescence devices, a protective layer and/or a protective cover is preferably provided to protect the device from the outside environment. Polymer compounds, metal oxides, metal fluorides and metal borides may be used to form the protective layers. The protective covers may be glass plates, plastic plates having a low water permeable surface, and metals. In a preferred embodiment, these covers are tightly laminated with the device substrates through a thermosetting resin or a photocurable resin. The use of spacers provides spaces in the packages and facilitates the prevention of scratches on the devices. Filling the spaces with inert gases such as nitrogen and argon prevents the oxidation of the cathodes. The placement of desiccants such as barium oxide in the spaces makes it easily possible to suppress water that has adsorbed on the devices during the manufacturing from causing damages to the devices. It is preferable that one or more of these approaches are adopted.

### (Substrates)

The substrates for the organic electroluminescence devices according to the present invention may be insulating substrates that are transparent to emission wavelengths of the emitting compounds. Known materials may be used, with examples including glass, transparent plastics such as PET (polyethylene terephthalates) and polycarbonates, and silicon substrates.

### (Constitutions of organic electroluminescence devices)

Fig. 1 is a cross-sectional view of an organic electroluminescence device according to an embodiment of the invention, in which a layer containing a hole transporting compound, an emitting compound and an electron transporting compound is interposed between an anode on a transparent substrate and a cathode.

The constitutions of the organic electroluminescence devices of the invention are not limited to the embodiment illustrated in Fig. 1.

### (Processes for producing organic electroluminescence devices)

The process for producing organic electroluminescence devices according to the invention includes a step of forming an organic layer on an anode by a coating method or a vapor deposition method, the organic layer containing a hole transporting compound having 4 to 8 arylamine structures in the molecule and a molecular weight in the range of 800 to 5000, an electron transporting compound having a structure of Formula (a) below and an emitting compound, and forming a cathode on the organic layer.

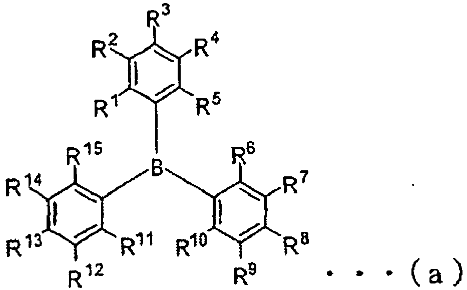

In Formula (a), R¹ to R¹⁵ are each independently a hydrogen atom, a halogen atom, a cyano group, an amino group, a C1-12 alkyl group, a C1-12 alkoxy group, an aryloxy group, an aryl group or a heterocyclic group, and neighboring substituent groups of R¹ to R¹⁵ that are on the same phenyl group may be bonded together to form a condensed ring.

Exemplary processes for producing organic electroluminescence devices of the invention will be described below. The processes of the invention, however, are not particularly limited thereto.

### (Coating method)

A substrate having an ITO anode (manufactured by NIPPO ELECTRIC CO., LTD.) is used. This substrate is a 25 mm square glass substrate in which two stripes of ITO (indium tin oxide) electrodes (anodes) are formed in a width of 4 mm on one surface of the substrate.

Poly(3,4-ethylenedioxythiophene) polystyrenesulfonic acid (Baytron P manufactured by Bayer AG) is spin coated on the ITO substrate at a rotation rate of 3500 rpm for an coating time of 40 seconds. The coating is dried in a vacuum drier at a reduced pressure and 60°C for 2 hours to form an anode buffer layer. Subsequently, a chloroform solution is prepared which contains a hole transporting compound at 48% by mass, an electron transporting compound at 49% by mass and an emitting compound at 3% by mass. The chloroform solution is spin coated on the anode buffer layer at a rotation rate of 3000 rpm for an coating time of 30 seconds. The coating is dried at room temperature (25°C) for 30 minutes to form an emitting layer. Thereafter, the substrate having the emitting layer is placed in a deposition apparatus, and Ba and Al are deposited to thicknesses of 10 nm and 100 nm, respectively, by vacuum deposition, thereby forming a cathode.

### (Vapor deposition method)

A substrate having an ITO anode (manufactured by NIPPO ELECTRIC CO., LTD.) is used. This substrate is a 25 mm square glass substrate in which two stripes of ITO (indium tin oxide) electrodes (anodes) are formed in a width of 4 mm on one surface of the substrate.

Poly(3,4-ethylenedioxythiophene) polystyrenesulfonic acid (Baytron P manufactured by Bayer AG) is spin coated on the ITO substrate at a rotation rate of 3500 rpm for an coating time of 40 seconds. The coating is dried in a vacuum drier at a reduced pressure and 60°C for 2 hours to form an anode buffer layer. Subsequently, a hole transporting compound, an electron transporting compound and an emitting compound are vacuum deposited to form an emitting layer which contains these compounds at 48% by mass, 49% by mass and 3% by mass, respectively. Thereafter, the substrate having the emitting layer is placed in a deposition apparatus, and Ba and Al are deposited to thicknesses of 10 nm and 100 nm, respectively, by vacuum deposition, thereby forming a cathode.

### (Uses)

The organic electroluminescence devices of the present invention are suitably used as pixels in matrix- or segment-type image display apparatuses according to conventional techniques. The organic EL devices are also suitable as surface-emitting light sources without forming pixels.

When the organic electroluminescence devices of the invention are designed to provide plane emission, a planar anode and cathode may be arranged so as to overlay each other. When patterned emission is desired, a mask having a patterned opening may be provided on the surface of the above planar emitting device, or portions of the organic layer that correspond to non-emitting parts may be formed in an extremely large thickness and thereby these portions may be rendered substantially non-emitting, or one or both of the anode and the cathode may be formed in a patterned shape. Display devices of segment type capable of displaying numbers, characters and simple symbols may be manufactured by creating patterns as described above and arranging the electrodes such that some electrodes can be turned on and off independently. Dot matrix devices may be manufactured by orthogonally arranging the stripe-shaped anodes and cathodes. Partial color displays or multicolor displays are possible by applying a plurality of organic electroluminescence compounds emitting different colors, or by using color filters or fluorescent conversion filters. The dot matrix devices may be passive matrix devices or active matrix devices in combination with TFTs or the like. These display devices may be used in computers, televisions, mobile terminals, cellular phones, car navigation systems and video camera viewfinders.

The planar emitting devices are self-emitting thin devices and are suitably used as planar backlight sources in liquid crystal display apparatuses or as planar illumination light sources. By adopting flexible substrates, the devices may be used as curved light sources or display devices.

### EXAMPLES

The present invention will be described in greater detail based on examples hereinbelow without limiting the scope of the invention.

### (DSC procedures)

The differential scanning calorimeter used was DSC 220 manufactured by Seiko Instruments Inc. A 5 mg sample was placed in an aluminum container and the container was tightly closed. The sample was heated from room temperature to 300°C at a temperature increasing rate of 10°C/min, held at the temperature for 5 minutes, and cooled to 30°C at a temperature decreasing rate of 10°C/min. Immediately thereafter, the sample was heated to 300°C at a rate of 10°C/min and then cooled to 30°C at a rate of 10°C/min, and this procedure was repeated two times.

### [Synthetic Examples]

Compounds 1 to 9 illustrated below were synthesized by methods described later.

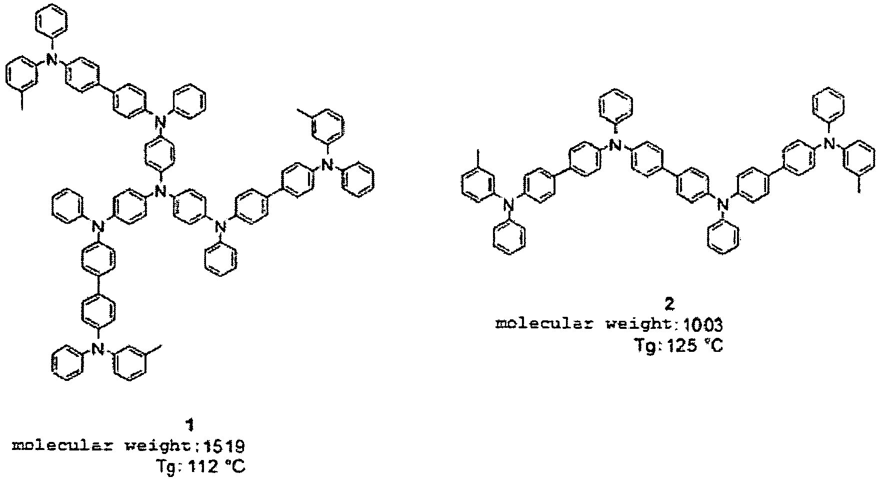

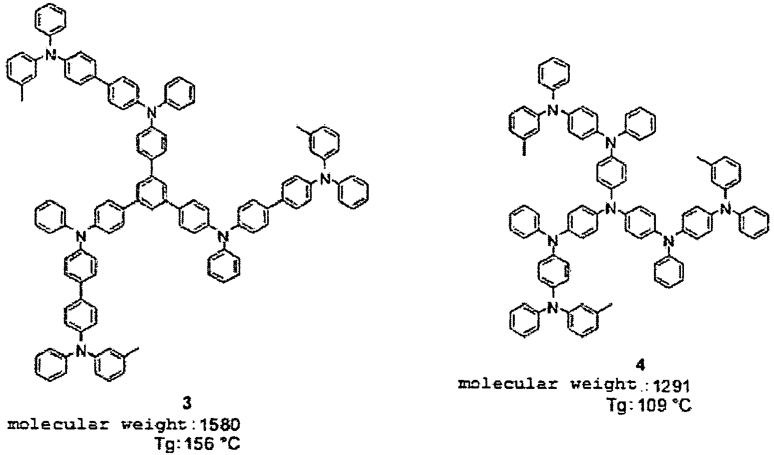

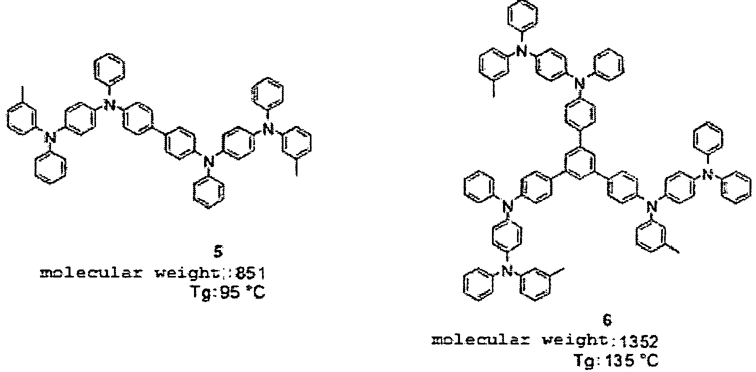

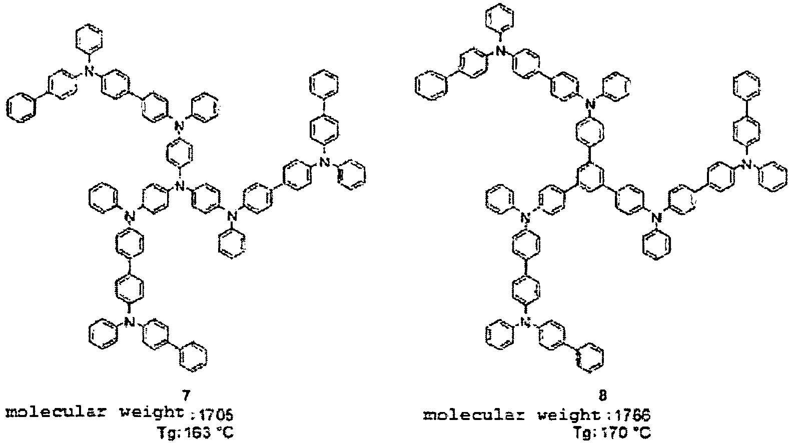

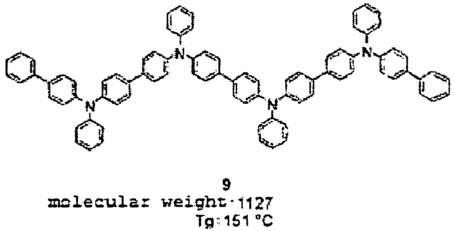

### Synthesis of N,N'-diphenyl-N-(m-tolyl)benzidine (10)

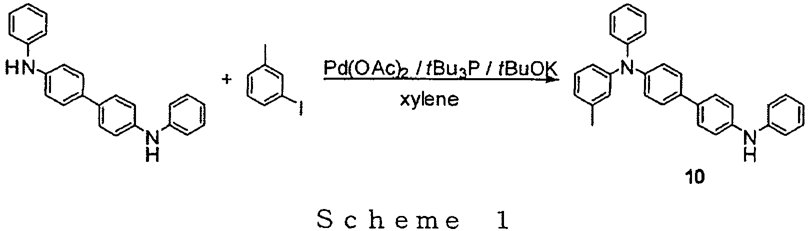

A 50 ml three-necked flask equipped with a condenser was charged with 1.346 g (4 mmol) of N,N'-diphenylbenzidine and was purged with nitrogen. Further, 20 ml of dry xylene and 4 mmol of m-iodotoluene were added, followed by stirring. Furthermore, 90 mg (0.4 mmol) of palladium acetate, 673 mg (6 mmol) of potassium-t-butoxide and 0.32 ml (0.8 mmol) of a 1:1 tri-t-butylphosphine:xylene solution were added, and the mixture was heated at 150°C for 6 hours with stirring.

After the reaction, the reaction solution was cooled to room temperature. Celite filtration was performed using a dichloromethane solvent. The solvent of the filtrate was evaporated under reduced pressure. The residue was purified by silica gel column chromatography (hexane:toluene = 1:1) to give 529 mg of N,N'-diphenyl-N-(m-tolyl) benzidine (10) (yield: 31%).

### Synthesis of compound 1

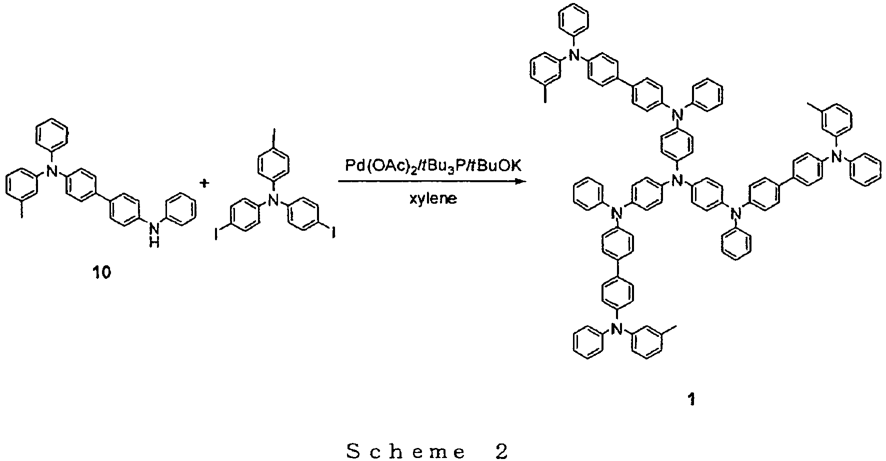

A 50 ml three-necked flask equipped with a condenser was charged with 427 mg (1 mmol) of the N,N'-diphenyl-N-(m-tolyl)benzidine (10) obtained above and 187 mg (0.3 mmol) of tris(4-iodophenyl)amine, and was purged with nitrogen. Further, 20 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:xylene solution were added, followed by stirring under reflux for 23 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 430 mg of compound 1 (yield: 94%).

### Synthesis of compound 2

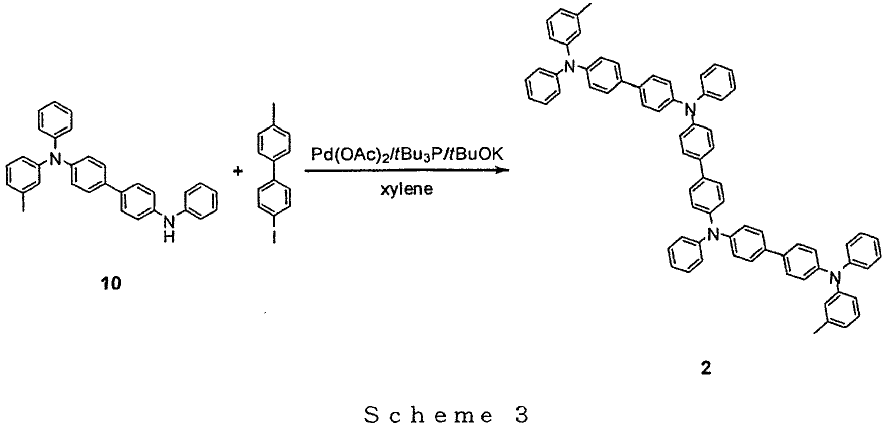

A 50 ml three-necked flask equipped with a condenser was charged with 427 mg (1 mmol) of the N,N'-diphehyl-N-(m-tolyl)benzidine (10) and 163 mg (0.4 mmol) of 4,4'-diiodobiphenyl, and was purged with nitrogen. Further, 10 ml of dry xylene, 23 mg (0.1 mmol) of palladium acetate, 168 mg (1.5 mmol) of potassium-t-butoxide and 0.1 ml (0.2 mmol) of a 1:1 tri-t-butylphosphine:toluene solution were added, followed by stirring under reflux for 23 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 500 mg of compound 2 (yield: 50%).

### Synthesis of compound 3

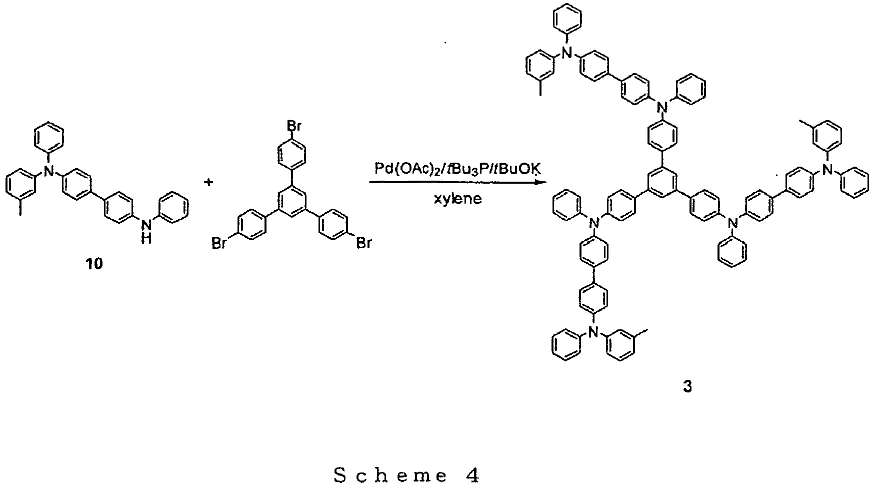

A 50 ml three-necked flask equipped with a condenser was charged with 427 mg (1 mmol) of the N,N'-diphenyl-N-(m-tolyl)benzidine (10) and 163 mg (0.3 mmol) of 1,3,5-tris(p-bromophenyl)benzene, and was purged with nitrogen. Further, 10 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 410 mg of compound 3 (yield: 86%).

### Synthesis of N,N'-diphenyl-N-(m-tolyl)-1,4-phenylenediamine (11)

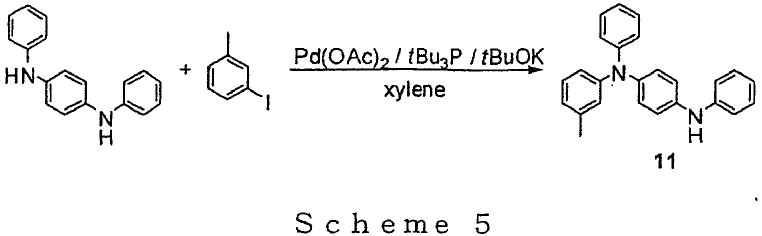

A 50 ml three-necked flask equipped with a condenser was charged with 2.603 g (10 mmol) of N,N'-diphenyl-1,4-phenylenediamine and was purged with nitrogen. Further, 30 ml of dry xylene and 1.710 g (10 mmol) of m-iodotoluene were added, followed by stirring. Furthermore, 225 mg (1 mmol) of palladium acetate, 1.683 g (15 mmol) of potassium-t-butoxide and 0.8 ml (2 mmol) of a 1:1 tri-t-butylphosphine:toluene solution were added, and the mixture was heated at 150°C for 7 hours with stirring.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 1.227 g of N,N'-diphenyl-N-(m-tolyl)-1,4-phenylenediamine (11) (yield: 35%).

### Synthesis of compound 4

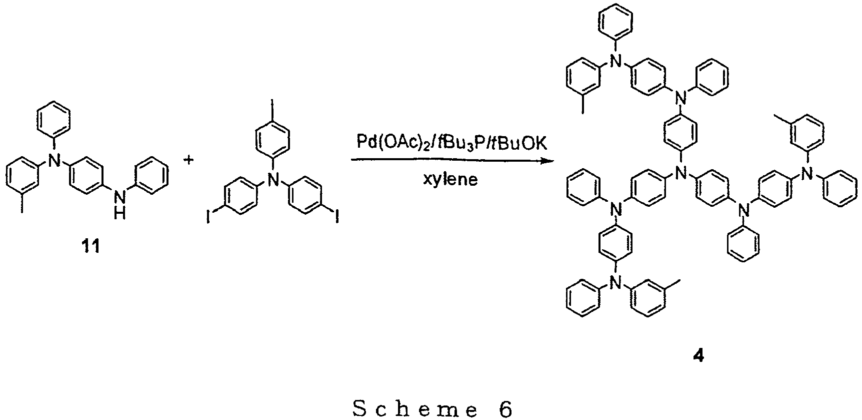

A 50 ml three-necked flask equipped with a condenser was charged with 351 mg (1 mmol) of the N,N'-diphenyl-N-(m-tolyl)-1,4-pheriylenediamine (11) and 187 mg (0.3 mmol) of tris(4-iodophenyl)amine, and was purged with nitrogen. Further, 20 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 430 mg of compound 4 (yield: 14%).

### Synthesis of compound 5

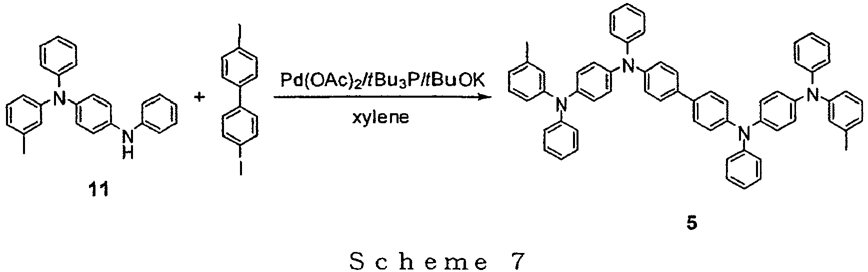

A 50 ml three-necked flask equipped with a condenser was charged with 351 mg (1 mmol) of the N,N'-diphenyl-N-(m-tolyl)-1,4-phenylenediamine (11) and 203 mg (0.5 mmol) of 4,4'-diiodobiphenyl, and was purged with nitrogen. Further, 10 ml of dry xylene, 23 mg (0.1 mmol) of palladium acetate, 168 mg (1.5 mmol) of potassium-t-butoxide and 0.1 ml (0.2 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 300 mg of compound 5 (yield: 65%).

### Synthesis of compound 6

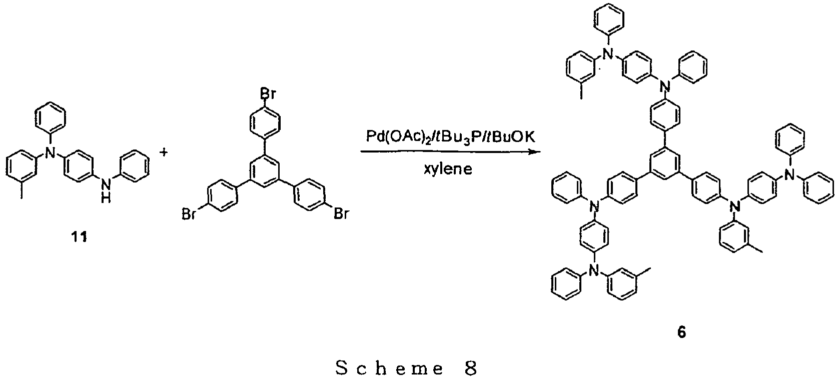

A 50 ml three-necked flask equipped with a condenser was charged with 351 mg (1 mmol) of the N,N'-diphenyl-N-(m-tolyl)-1,4-phenylenediamine (11) and 163 mg (0.3 mmol) of 1,3,5-tris(p-bromophenyl)benzene, and was purged with nitrogen. Further, 10 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 178 mg of compound 6 (yield: 44%).

### Synthesis of N,N'-diphenyl-N-biphenylbenzidine (12)

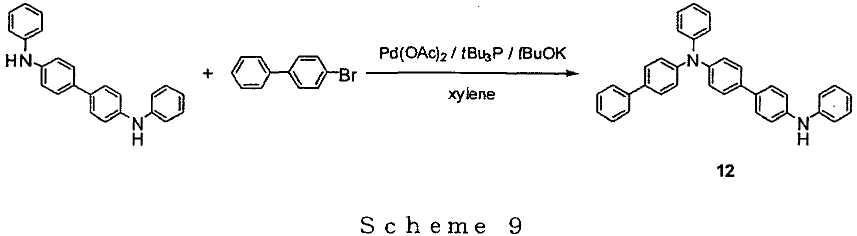

A 50 ml three-necked flask equipped with a condenser was charged with 3.364 g (10 mmol) of N,N'-diphenylbenzidine and 2.331 g (10 mmol) of 4-bromobiphenyl, and was purged with nitrogen. Further, 30 ml of dry xylene was added, followed by stirring. Furthermore, 225 mg (1 mmol) of palladium acetate, 1.683 g (15 mmol) of potassium-t-butoxide and 0.8 ml (2 mmol) of a 1:1 tri-t-butylphosphine:xylene solution were added, and the mixture was heated at 160°C for 24 hours with stirring.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) to give 1.37 g of N,N'-diphenyl-N-biphenylbenzidine (12) (yield: 28%).

### Synthesis of compound 7

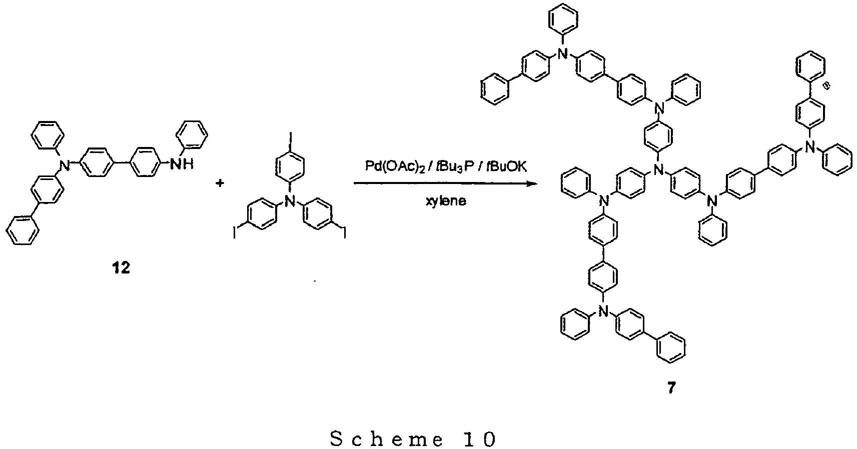

A 50 ml three-necked flask equipped with a condenser was charged with 351 mg (1 mmol) of the N,N'-diphenyl-N-biphenylbenzidine (12) and 187 mg (0.3 mmol) of tris(4-iodophenyl)amine, and was purged with nitrogen. Further, 10 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. Purification of the residue was attempted by silica gel column chromatography (hexane:toluene = 1:1), but the residue appeared to be crystallized in the column probably because of low solubility. Toluene was then passed through the column to recover the components that eluted. The solvent was evaporated under reduced pressure, and the residue was subjected to reprecipitation with toluene and acetone to afford 322 mg of compound 7 (yield: 63%).

### Synthesis of compound 8

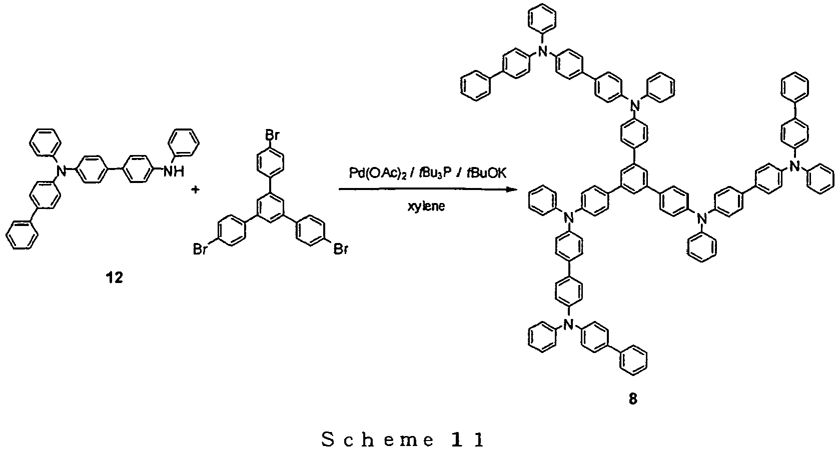

A 50 ml three-necked flask equipped with a condenser was charged with 351 mg (1 mmol) of the N,N'-diphenyl-N-biphenylbenzidine (12) and 163 mg (0.3 mmol) of 1,3,5-tris(p-bromophenyl)benzene, and was purged with nitrogen. Further, 10 ml of dry xylene, 34 mg (0.15 mmol) of palladium acetate, 168 mg (1.5 mmol) of potassium-t-butoxide and 0.12 ml (0.3 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. Purification of the residue was attempted by silica gel column chromatography (hexane:toluene = 1:1), but the residue appeared to be crystallized in the column similar to the compound (7). Toluene was then passed through the column to recover the components that eluted. The solvent was evaporated under reduced pressure, and the residue was subjected to reprecipitation with toluene and acetone to afford 381 mg of compound 8 (yield: 72%).

### Synthesis of compound 9

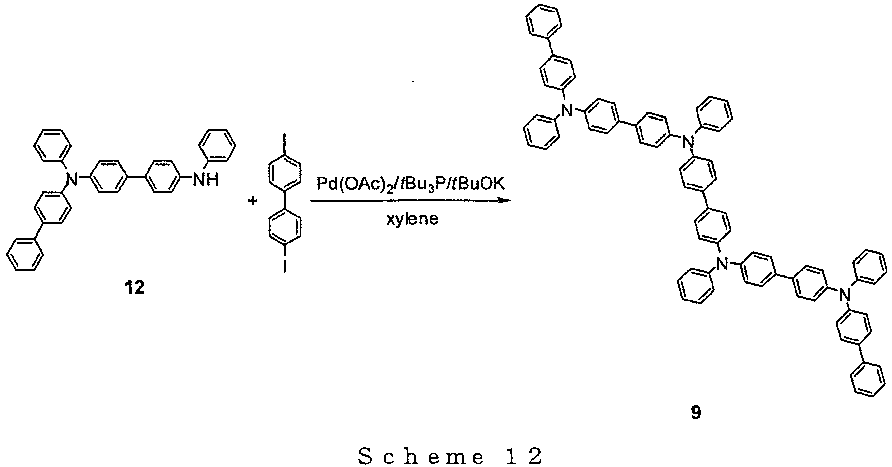

A 50 ml three-necked flask equipped with a condenser was charged with 293 mg (0.6 mmol) of the N,N'-diphenyl-N-biphenylbenzidine (12) and 102 mg (0.25 mmol) of 4,4' -diiodobiphenyl, and was purged with nitrogen. Further, 10 ml of dry xylene, 23 mg (0.1 mmol) of palladium acetate, 112 mg (1 mmol) of potassium-t-butoxide and 0.08 ml (0.2 mmol) of a 1:1 tri-t-butylphosphine:toluene solution were added, followed by stirring under reflux for 24 hours.

After the reaction, the reaction mixture was cooled to room temperature. Celite filtration was performed using a dichloromethane solvent. The solvent of the filtrate was evaporated under reduced pressure. The residue was purified by silica gel column chromatography (hexane:toluene = 1:1) and was subjected to reprecipitation with toluene and acetone to give 259 mg of compound 9 (yield: 92%).

### [Example 1]

### (Fabrication of organic EL devices and evaluation of electroluminescence properties)

Organic EL devices were fabricated using a 25 mm square, ITO (indium tin oxide) glass substrate (manufactured by NIPPO ELECTRIC CO., LTD.) in which two stripes of ITO electrodes were formed as anodes in a width of 4 mm on one surface of the substrate.

First, poly(3,4-ethylenedioxythiophene) polystyrenesulfonic acid (Baytron P manufactured by Bayer AG) was spin coated on the ITO electrodes (anodes) of the ITO-coated substrate at a rotation rate of 3500 rpm for an coating time of 40 seconds. The coating was dried in a vacuum drier at a reduced pressure and 60°C for 2 hours to form anode buffer layers. The thickness of the anode buffer layers was approximately 50 nm.

Subsequently, a coating solution for forming an emitting layer was prepared by dissolving 48 mg of the aforesaid compound 1 and 49 mg and 3 mg of compounds ETM-3 and E-32, respectively, illustrated below in 2900 mg of chloroform (special grade, manufactured by Wako Pure Chemical Industries, Ltd.), and filtering the resultant solution through a 0.2 µm pore filter.

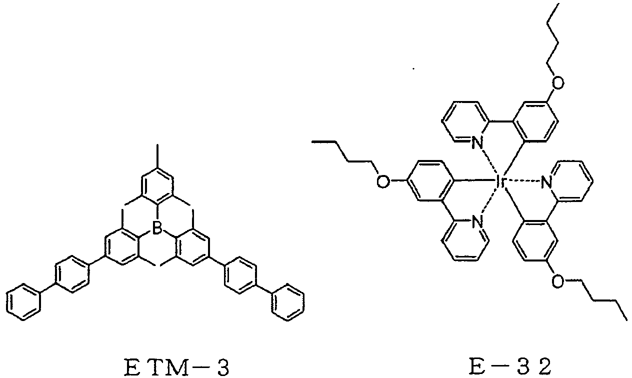

Thereafter, the coating solution was spin coated on the anode buffer layers at a rotation rate of 3000 rpm for an coating time of 30 seconds. After the coating, the coating was dried at room temperature (25°C) for 30 minutes to form emitting layers. The thickness of the emitting layers was approximately 100 nm. Subsequently, the substrate having the emitting layers was placed in a vacuum deposition apparatus. Cesium was deposited to a thickness of 2 nm at a deposition rate of 0.01 nm/s (an alkali metal dispenser manufactured by SAES Getters was used as a cesium source), and aluminum as cathode was deposited to a thickness of 250 nm at a deposition rate of 1 nm/s, thereby forming devices 1. The above cesium and aluminum layers were formed in two stripes with a width of 3 mm that were perpendicular to the anode-extending direction, and thereby four organic EL devices 4 mm in length and 3 mm in width were fabricated on the glass substrate.

The organic EL devices were caused to emit light by applying a voltage thereto by means of programmable direct-current voltage/current source TR6143 manufactured by ADVANTEST CORPORATION. The luminance was measured with luminance meter BM-8 manufactured by TOPCON CORPORATION. Table 2 shows the external quantum efficiency at 100 cd/m² and luminance half life from the initial luminance of 1000 cd/m² (the values are averages of the four devices fabricated on the substrate).

### [Examples 2 to 13 and Comparative Examples 1 to 5]

Devices 2 to 18 (Examples 2 to 13 and Comparative Examples 1 to 5) were fabricated in the same manner as in Example 1, except that the compound 1: 48 mg, ETM-3: 49 mg and E-32: 3 mg were changed to materials shown in Table 1. The devices were tested as described in Example 1 to evaluate electroluminescence properties, the results being set forth in Table 2. In Table 1, ETM-1 to ETM-5, TBA, TCTA, PBD, OXD7 and TAZ are the compounds illustrated below.

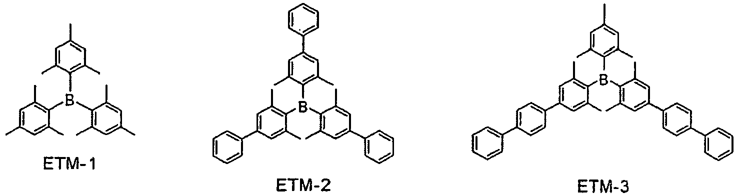

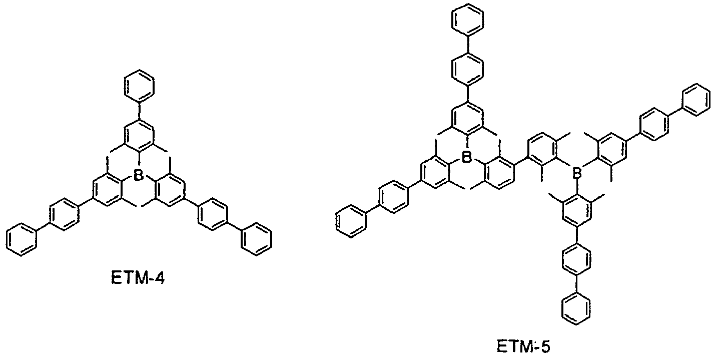

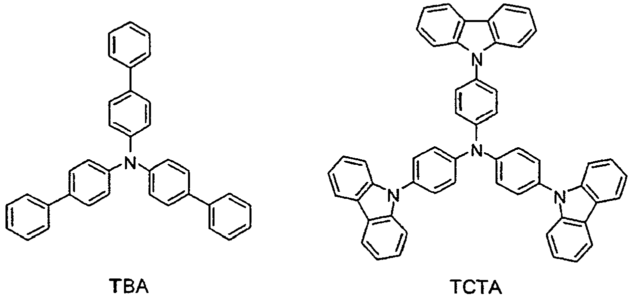

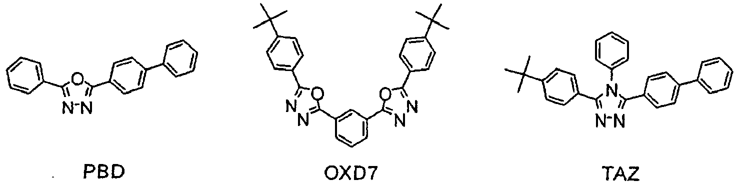

**Table 1**

| Device No. | Materials |
|---|---|
| 1 (Ex. 1) | Compound 1: 48 mg, ETM-3: 49 mg, E-32: 3 mg |
| 2 (Ex. 2) | Compound 2: 48 mg, ETM-3: 49 mg, E-32: 3 mg |
| 3 (Ex. 3) | Compound 3: 48 mg, ETM-3: 49 mg, E-32: 3 mg |
| 4 (Ex. 4) | Compound 4: 48 mg, ETM-3: 49 mg, E-32: 3 mg |
| 5 (Ex. 5) | Compound 5: 48 mg, ETM-3: 49 mg, E-32: 3 mg |
| 6 (Ex. 6) | Compound 6: 48 mg, ETM-3: 49 mg, E-32: 3 mg |
| 7 (Ex. 7) | Compound 7: 48 mg, ETM-3: 49 mg, E-32: 3 mg |
| 8 (Ex. 8) | Compound 8: 48 mg, ETM-3: 49 mg, E-32: 3 mg |
| 9 (Ex. 9) | Compound 9: 48 mg, ETM-3: 49 mg, E-32: 3 mg |
| 10 (Ex. 10) | Compound 1: 48 mg, ETM-2: 49 mg, E-32: 3 mg |
| 11 (Ex. 11) | Compound 1: 48 mg, ETM-4: 49 mg, E-32: 3 mg |
| 12 (Ex. 12) | Compound 1: 48 mg, ETM-1: 49 mg, E-32: 3 mg |
| 13 (Ex. 13) | Compound 1: 48 mg, ETM-5: 49 mg, E-32: 3 mg |
| 14 (Comp. Ex. 1) | TBA: 48 mg, ETM-3: 49 mg, E-32: 3 mg |
| 15 (Comp. Ex. 2) | TCTA: 48 mg, ETM-3: 49 mg, E-32: 3 mg |
| 16 (Comp. Ex. 3) | Compound 1: 48 mg, PBD: 49 mg, E-32: 3 mg |
| 17 (Comp. Ex. 4) | Compound 1: 48 mg, OXD7: 49 mg, E-32: 3 mg |
| 18 (Comp. Ex. 5) | Compound 1: 48 mg, TAZ: 49 mg, E-32: 3 mg |

**Table 2**

| Device No. | External quantum efficiency (%) | Durability (relative values at 1000 cd/m²) |
|---|---|---|
| 1 (Ex. 1) | 7.3 | 2700 |
| 2 (Ex. 2) | 5.0 | 270 |
| 3 (Ex. 3) | 8.3 | 2700 |
| 4 (Ex. 4) | 6.0 | 1000 |
| 5 (Ex. 5) | 5.2 | 1000 |
| 6 (Ex. 6) | 5.4 | 800 |
| 7 (Ex. 7) | 5.1 | 400 |
| 8 (Ex. 8) | 6.4 | 1400 |
| 9 (Ex. 9) | 7.3 | 2500 |
| 10 (Ex. 10) | 7.9 | 2100 |
| 11 (Ex. 11) | 6.7 | 2100 |
| 12 (Ex. 12) | 8.2 | 1000 |
| 13 (Ex. 13) | 7.2 | 3200 |
| 14 (Comp. Ex. 1) | Not available because of a short-circuit failure in the device | |
| 15 (Comp. Ex. 2) | Not available because of a short-circuit failure in the device | |
| 16 (Comp. Ex. 3) | 3.2 | 100 |
| 17 (Comp. Ex. 4) | 4.3 | 80 |
| 18 (Comp. Ex. 5) | 3.4 | 50 |

| | | |
|---|---|---|
| *Durability indicates values relative to the half life in Comparative Example 3 (Compound 1: 48 mg, PBD: 49 mg, E-32: 3 mg) as 100. | | |

### [Example 14]

### (Fabrication of organic EL devices and evaluation of electroluminescence properties)

Organic EL devices were fabricated using a 25 mm square, ITO (indium tin oxide) glass substrate (manufactured by NIPPO ELECTRIC CO., LTD.) in which two stripes of ITO electrodes were formed as anodes in a width of 4 mm on one surface of the substrate. First, poly(3,4-ethylenedioxythiophene) polystyrenesulfonic acid (Baytron P manufactured by Bayer AG) was spin coated on the ITO electrodes (anodes) of the ITO-coated substrate at a rotation rate of 3500 rpm for an coating time of 40 seconds. The coating was dried in a vacuum drier at a reduced pressure and 60°C for 2 hours to form anode buffer layers. The thickness of the anode buffer layers was approximately 50 nm.

Subsequently, the substrate was placed in a vacuum deposition apparatus in order to form emitting layers thereon. The compound 1, ETM-3 and E-32 were co-deposited to a thickness of 100 nm at a deposition rate of 0. 5 nm/s such that the resultant emitting layers contained these compounds at 48% by mass, 49% by mass and 3% by mass, respectively. The substrate having the emitting layers was then placed in a vacuum deposition apparatus. Cesium was deposited to a thickness of 2 nm at a deposition rate of 0.01 nm/s (an alkali metal dispenser manufactured by SAES Getters was used as a cesium source), and aluminum as cathode was deposited to a thickness of 250 nm at a deposition rate of 1 nm/s, thereby forming devices 21. The above cesium and aluminum layers were formed in two stripes with a width of 3 mm that were perpendicular to the anode-extending direction, and thereby four organic EL devices 4 mm in length and 3 mm in width were fabricated on the glass substrate.

The organic EL devices were caused to emit light by applying a voltage thereto by means of programmable direct-current voltage/current source TR6143 manufactured by ADVANTEST CORPORATION. The luminance was measured with luminance meter BM-8 manufactured by TOPCON CORPORATION. Table 4 shows the external quantum efficiency at 100 cd/m² and luminance half life from the initial luminance of 1000 cd/m² (the values are averages of the four devices fabricated on the substrate).

### [Examples 15 to 22 and Comparative Examples 6 to 8]

Devices 19 to 30 (Examples 15 to 22 and Comparative Examples 6 to 8) were manufactured in the same manner as in Example 14, except that the compound 1: 48% by mass, ETM-3: 49% by mass and E-32: 3% by mass were changed to materials shown in Table 3. The devices were tested as described in Example 14 to evaluate electroluminescence properties, the results being set forth in Table 4.

**Table 3**

| Device No. | Materials |
|---|---|
| 19 (Ex. 14) | Compound 1: 48% by mass, ETM-3: 49% by mass, E-32: 3% by mass |
| 20 (Ex. 15) | Compound 2: 48% by mass, ETM-3: 49% by mass, E-32: 3% by masts |
| 21 (Ex. 16) | Compound 3: 48% by mass, ETM-3: 49% by mass, E-32: 3% by mass |
| 22 (Ex. 17) | Compound 4: 48% by mass, ETM-3: 49% by mass, E-32: 3% by mass |
| 23 (Ex. 18) | Compound 5: 48% by mass, ETM-3: 49% by mass, E-32: 3% by mass |
| 24 (Ex. 19) | Compound 6: 48% by mass, ETM-3: 49% by mass, E-32: 3% by mass |
| 25 (Ex. 20) | Compound 7: 48% by mass, ETM-3: 49% by mass, E-32: 3% by mass |
| 26 (Ex. 21) | Compound 8: 48% by mass, ETM-3: 49% by mass, E-32: 3% by mass |
| 27 (Ex. 22) | Compound 9: 48% by mass, ETM-3: 49% by mass, E-32: 3% by mass |
| 28 (Comp. Ex. 6) | TBA: 48% by mass, ETM-3: 49% by mass, E-32: 3% by mass |
| 29 (Comp. Ex. 7) | TCTA: 48% by mass, ETM-3: 49% by mass, E-32: 3% by mass |
| 30 (Comp. Ex. 8) | Compound 1: 48% by mass, PBD: 49% by mass, E-32: 3% by mass |

**Table 4**

| Device No. | External quantum efficiency (%) | Durability (relative values at 1000 cd/m²) |
|---|---|---|
| 19 (Ex. 14) | 7.0 | 2400 |
| 20 (Ex. 15) | 5.0 | 260 |
| 21 (Ex. 16) | 8.1 | 2400 |
| 22 (Ex. 17) | 6.3 | 800 |
| 23 (Ex. 18) | 5.0 | 700 |
| 24 (Ex. 19) | 5.5 | 800 |
| 25 (Ex. 20) | 5.3 | 500 |
| 26 (Ex. 21) | 6.5 | 1200 |
| 27 (Ex. 22) | 7.0 | 2000 |
| 28 (Comp. Ex. 6) | 4.9 | 70 |
| 29 (Comp. Ex. 7) | 2.8 | 130 |
| 30 (Comp. Ex. 8) | 3.1 | 100 |

| | | |
|---|---|---|
| *Durability indicates values relative to the half life in Comparative Example 8 (Compound 1: 48% by mass, PBD: 49% by mass, E-32: 3% by mass) as 100. | | |

## Claims

1. An organic electroluminescence device comprising a pair of electrodes and one or more organic layers including an emitting layer between the pair of electrodes, wherein one of the organic layer (s) comprises electron transporting compound having a structure of Formula (a) below and an emitting compound; 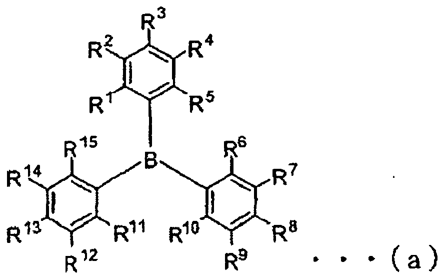 wherein R¹ to R¹⁵ are each independently a hydrogen atom, a halogen atom, a cyano group, an amino group, a C1-12 alkyl group, a C1-12 alkoxy group, an aryloxy group, an aryl group or a heterocyclic group, and neighboring substituent groups of R¹ to R¹⁵ that are on the same phenyl group may be bonded together to form a condensed ring; and **characterized in that** said organic layer further comprises a hole transporting compound having 4 to 8 arylamine structures in the molecule and a molecular weight in the range of 800 to 5000.

2. The organic electroluminescence device according to claim 1, wherein the hole transporting compound has 10 or more benzene ring structures in the molecule.

3. The organic electroluminescence device according to claim 1 or 2, wherein the hole transporting compound has a glass transition temperature Tg in the range of 80 to 300°C.

4. The organic electroluminescence device according to any one of claims 1 to 3, wherein the hole transporting compound is at least one selected from compounds 1 to 9 illustrated below:

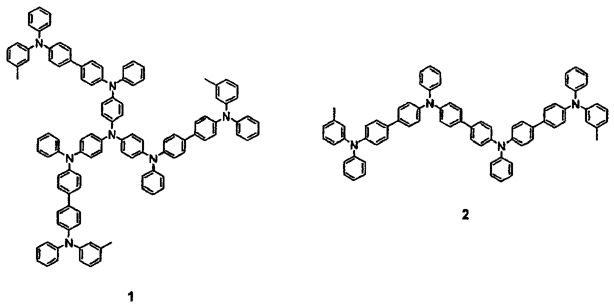

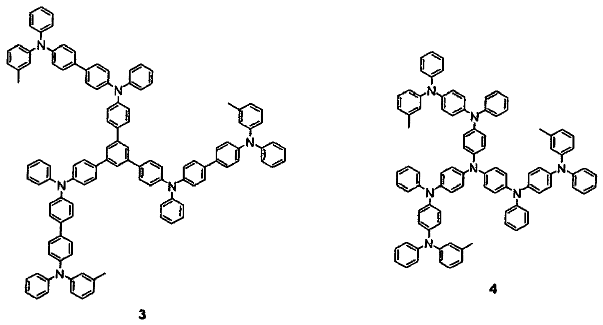

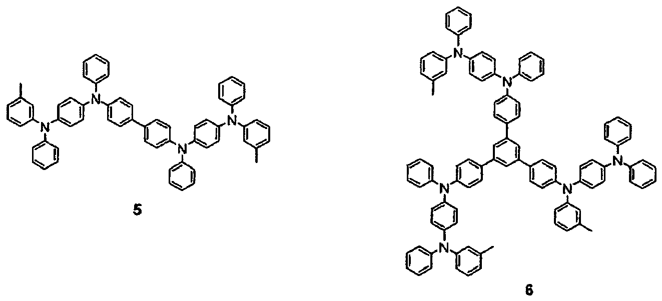

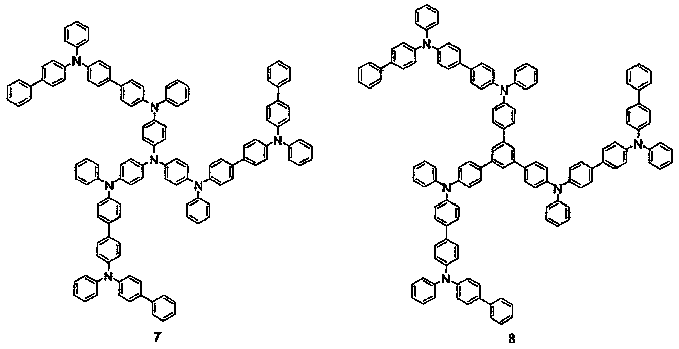

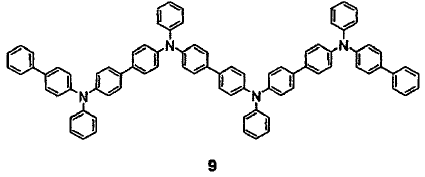

5. A process for producing organic electroluminescence devices, comprising a step of forming an organic layer on an anode, the organic layer comprising an electron transporting compound having a structure of Formula (a) below and an emitting compound, and forming a cathode on the organic layer; 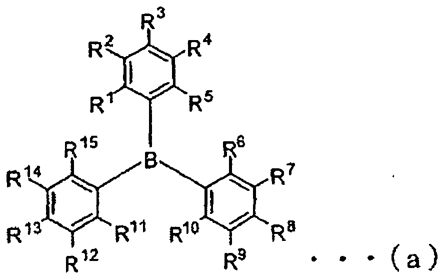 wherein R¹ to R¹⁵ are each independently a hydrogen atom, a halogen atom, a cyano group, an amino group, a C1-12 alkyl group, a C1-12 alkoxy group, an aryloxy group, an aryl group or a heterocyclic group, and neighboring substituent groups of R¹ to R¹⁵ that are on the same phenyl group may be bonded together to form a condensed ring; and **characterized in that** said organic layer further comprises a hole transporting compound having 4 to 8 arylamine structures in the molecule and a molecular weight in the range of 800 to 5000.

6. An image display apparatus comprising the organic electroluminescence device described in any one of claims 1 to 4.

7. A surface-emitting light source comprising the organic electroluminescence device described in any one of claims 1 to 4.

## Patentansprüche

1. Organische Elektrolumineszenzvorrichtung, umfassend ein Elektrodenpaar und eine oder mehrere organische Schichten, die eine emittierende Schicht zwischen dem Elektrodenpaar aufweisen, wobei eine der organischen Schichten folgendes umfasst:
eine Elektronen leitende Verbindung mit einer Struktur der Formel (a) unten und eine emittierende Verbindung: 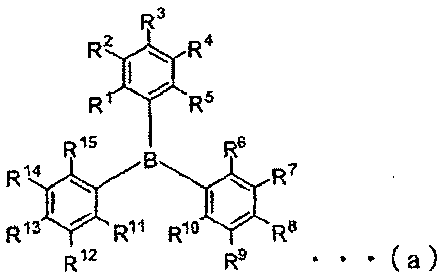 wobei R¹ bis R¹⁵ jeweils unabhängig ein Wasserstoffatom, ein Halogenatom, eine Cyanogruppe, eine Aminogruppe, eine C1-C12-Alkylgruppe, eine C1-C12-Alkoxygruppe, eine Aryloxygruppe, eine Arylgruppe oder eine heterocyclische Gruppe sind, und wobei benachbarte Substituentengruppen von R¹ bis R¹⁵, die sich in der gleichen Phenylgruppe befinden, miteinander verbunden sein können, um einen kondensierten Ring zu bilden; und **dadurch gekennzeichnet, dass** die organische Schicht ferner eine Lochleitungsverbindung mit 4 bis 8 Arylaminstrukturen in dem Molekül und einem Molekulargewicht im Bereich von 800 bis 5000 umfasst.

2. Organische Elektrolumineszenzvorrichtung nach Anspruch 1, wobei die Lochleitungsverbindung 10 oder mehr Benzolringstrukturen in dem Molekül aufweist.

3. Organische Elektrolumineszenzvorrichtung nach Anspruch 1 oder 2, wobei die Lochleitungsverbindung eine Glasübergangstemperatur Tg im Bereich von 80 bis 300 °C aufweist.

4. Organische Elektrolumineszenzvorrichtung nach einem der Ansprüche 1 bis 3, wobei die Lochleitungsverbindung wenigstens eine aus den nachstehend veranschaulichten Verbindungen 1 bis 9 ausgewählte Verbindung ist:

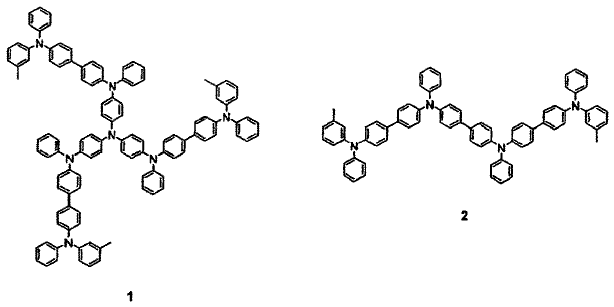

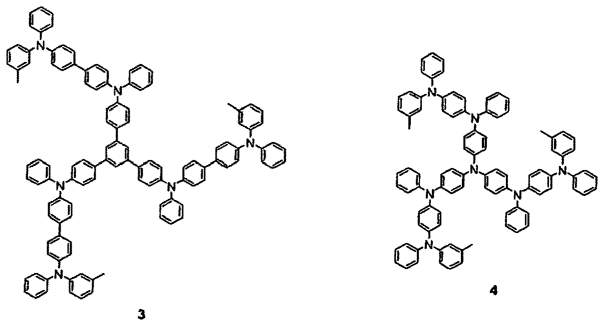

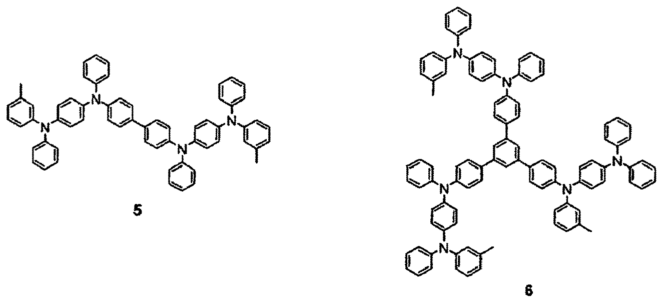

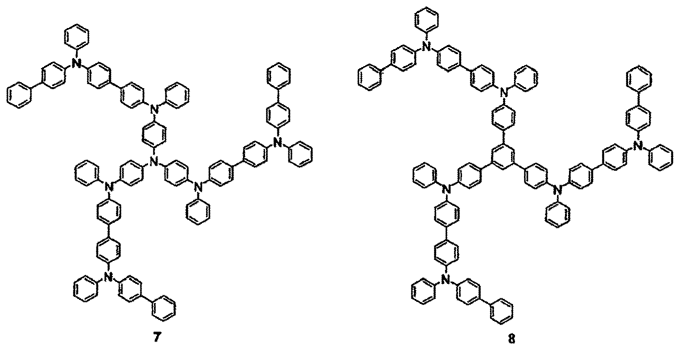

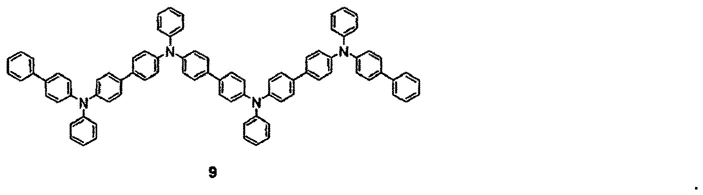

5. Verfahren zum Herstellen organischer Elektrolumineszenzvorrichtungen, umfassend den Schritt des Ausbildens einer organischen Schicht an einer Anode, wobei die organische Schicht folgendes umfasst:
eine Elektronen leitende Verbindung mit einer Struktur der Formel (a) unten und eine emittierende Verbindung, und das Ausbilden einer Kathode auf der organischen Schicht: 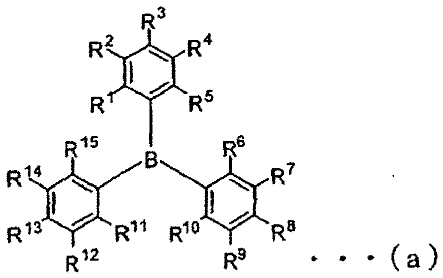 wobei R¹ bis R¹⁵ jeweils unabhängig ein Wasserstoffatom, ein Halogenatom, eine Cyanogruppe, eine Aminogruppe, eine C1-C12-Alkylgruppe, eine C1-C12-Alkoxygruppe, eine Aryloxygruppe, eine Arylgruppe oder eine heterocyclische Gruppe sind, und wobei benachbarte Substituentengruppen von R¹ bis R¹⁵, die sich in der gleichen Phenylgruppe befinden, miteinander verbunden sein können, um einen kondensierten Ring zu bilden; und **dadurch gekennzeichnet, dass** die organische Schicht ferner eine Lochleitungsverbindung mit 4 bis 8 Arylaminstrukturen in dem Molekül und einem Molekulargewicht im Bereich von 800 bis 5000 umfasst.

6. Bildanzeigevorrichtung, umfassend die organische Elektrolumineszenzvorrichtung gemäß einem der Ansprüche 1 bis 4.

7. Flächenemittierende Lichtquelle, umfassend die die organische Elektrolumineszenzvorrichtung gemäß einem der Ansprüche 1 bis 4.

## Revendications

1. Dispositif électroluminescent organique comprenant une paire d'électrodes et une ou plusieurs couches organiques comprenant une couche émettrice entre la paire d'électrodes, l'une de la ou des couches organiques comprenant un composé de transport d'électron ayant une structure de formule (a) ci-dessous et un composé émetteur ; 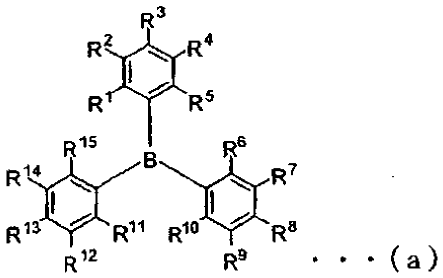 R¹ à R¹⁵ étant chacun indépendamment un atome d'hydrogène, un atome d'halogène, un groupe cyano, un groupe amino, un groupe alkyle en C₁ à C₁₂, un groupe alcoxy en C₁ à C₁₂, un groupe aryloxy, un groupe aryle ou un groupe hétérocyclique, et les groupes substituants voisins de R¹ à R¹⁵ qui se trouvent sur le même groupe phényl pouvant être collés ensemble pour former un anneau condensé ; et **caractérisé en ce que** ladite couche organique comprend en outre un composé de transport de trou comportant de 4 à 8 structures d'arylamine dans la molécule et un poids moléculaire compris entre 800 et 5 000.

2. Dispositif électroluminescent organique selon la revendication 1, le composé de transport de trou ayant 10 structures à cycle benzénique ou plus dans la molécule.

3. Dispositif électroluminescent organique selon la revendication 1 ou 2, le composé de transport de trou ayant une température de transition vitreuse Tg comprise entre 80 et 300 °C.

4. Dispositif électroluminescent organique selon l'une quelconque des revendications 1 à 3, le composé de transport de trou étant au moins choisi parmi les composés 1 à 9 illustrés ci-dessous :

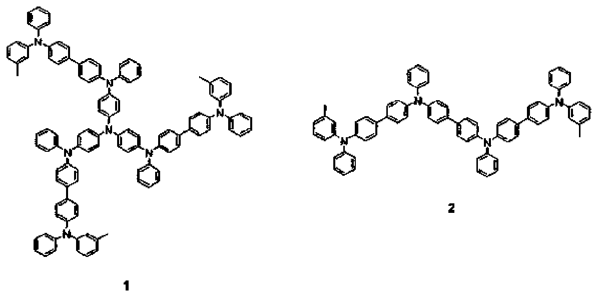

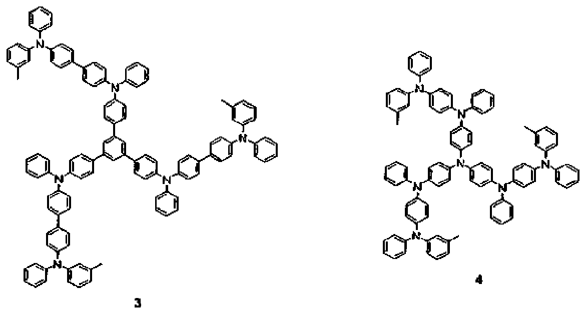

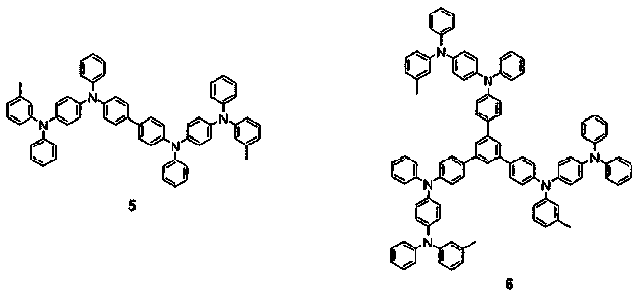

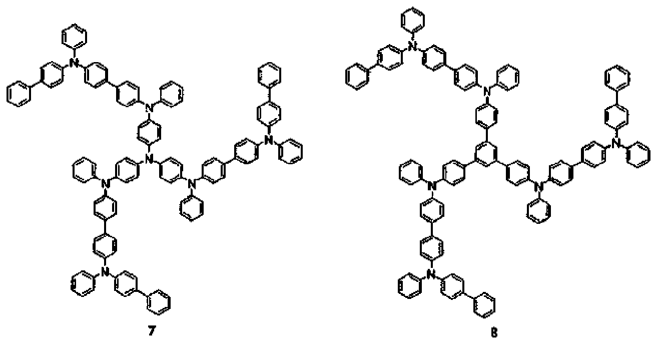

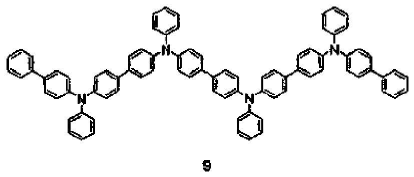

5. Procédé de fabrication de dispositifs électroluminescents organiques, comprenant les étapes consistant à former une couche organique sur une anode, la couche organique comprenant un composé de transport d'électron ayant une structure de formule (a) ci-dessous et un composé émetteur, et former une cathode sur la couche organique ; 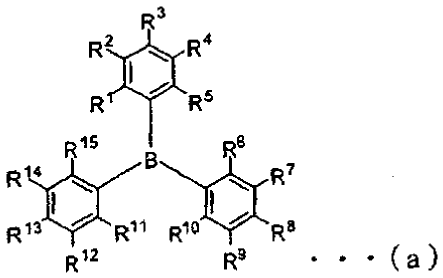 R¹ à R¹⁵ étant chacun indépendamment un atome d'hydrogène, un atome d'halogène, un groupe cyano, un groupe amino, un groupe alkyle en C₁ à C₁₂, un groupe alcoxy en C₁ à C₁₂, un groupe aryloxy, un groupe aryle ou un groupe hétérocyclique, et les groupes substituants voisins de R1 à R15 qui se trouvent sur le même groupe phényl pouvant être collés ensemble pour former un anneau condensé ; et **caractérisé en ce que** ladite couche organique comprend en outre un composé de transport de trou comportant de 4 à 8 structures d'arylamine dans la molécule et un poids moléculaire compris entre 800 et 5 000.

6. Appareil d'affichage d'image comprenant le dispositif électroluminescent organique selon l'une quelconque des revendications 1 à 4.

7. Source électroluminescente de surface comprenant le dispositif électroluminescent organique selon l'une quelconque des revendications 1 à 4.
